# EUROPÄISCHE PATENTSCHRIFT

(11) **EP 1 066 393 B1**
(45) Veröffentlichungstag und Bekanntmachung des Hinweises auf die Patenterteilung: **03.07.2002**
(21) Anmeldenummer: 99917949.2
(22) Anmeldetag: 01.04.1999
(51) Int. Cl.: C12N 15/80, C12N 9/02, C12N 15/12

(54) **EXPRESSIONSSYSTEM ZUR PRODUKTION VON PROTEINEN**
EXPRESSION SYSTEM FOR PRODUCING PROTEINS
SYSTEME D'EXPRESSION POUR LA PRODUCTION DE PROTEINES

(30) Priorität: 02.04.1998 DE 19814853
(43) Veröffentlichungstag der Anmeldung: 10.01.2001
(73) Patentinhaber: Consortium für elektrochemische Industrie GmbH, 81379 München (DE)
(72) Erfinder: PFALLER, Rupert, D-80639 München (DE); HESSING, Johanna, NL-2624 PG Delft (NL); VAN DEN HONDEL, Cornelis, NL-2804 PZ Gouda (NL); VAN GORCOM, Robertus, NL-3768 GW Soest (NL)
(74) Vertreter: Potten, Holger
(86) Internationale Anmeldenummer: EP9902252
(87) Internationale Veröffentlichungsnummer: WO9951757

(56) Entgegenhaltungen:
- WO-A-93/12259
- WO-A-96/00290
- PATENT ABSTRACTS OF JAPAN vol. 097, no. 006, 30. Juni 1997 (1997-06-30) & JP 09 047289 A (OJI PAPER CO LTD), 18. Februar 1997 (1997-02-18)

## Beschreibung

Die Erfindung betrifft ein Expressionssystem zur Produktion von Proteinen in Pilzen der Gattungen Trametes oder Polyporus, seine Herstellung und seine Verwendung.

Zur Proteinproduktion sind verschiedene prokaryontische und eukaryontische Expressionssysteme bekannt. Beispiele prokaryontischer Expressionssysteme sind Escherichia coli und Bacillus subtilis. Die Methoden zur genetischen Manipulation dieser Organismen sind wohl etabliert. Spezifische Nachteile dieser Expressionssysteme sind die oft enttäuschend niedrige Produktionsrate vor allem eukaryontischer Proteine, die Faltung der produzierten Proteine derart, daß sie häufig nicht in aktiver Form vorliegen sowie vor allem auch das Fehlen der posttranslationalen Modifikation der exprimierten Proteine. Als fehlende posttranslationale Modifikation seien beispielsweise der fehlende Einbau prosthetischer Gruppen oder die fehlende Glykosylierung des zu exprimierenden Proteins genannt.

Diese Nachteile prokaryontischer Expressionssysteme können durch Verwendung eukaryontischer Expressionssysteme umgangen werden.

Zu den verbreiteten eukaryontischen Expressionssystemen mit breiter Anwendung gehören Zellkultursysteme sowohl von Säugerzellen wie auch Insektenzellen sowie eukaryontische Mikroorganismen wie Hefen oder filamentöse Pilze. Während bei diesen Expressionssystemen das zu exprimierende Protein in der Regel in aktiver Form gebildet wird, ist die Produktionsrate vor allem bei der Expression heterologer Proteine in vielen Fällen zu niedrig. Als Beispiel hierfür kann die Expression in der Hefe Saccharomyces cerevisiae oder in filamentösen Pilzen aus der Klasse der Ascomyceten dienen.

Hohe Produktionsraten in filamentösen Pilzen wie Aspergillus sind vor allem für die Expression homologer Proteine oder Proteine von filamentösen Pilzen der Klasse der Ascomyceten beschrieben. Die Expression heterologer Proteine gelingt oft nur mit geringen oder mäßigen Ausbeuten. WO 96/00290 z. B. beschreibt die heterologe Expression der Laccase LCC1 aus dem filamentösen Pilz der Klasse der Basidiomyceten, Polyporus pinsitus. Bei der Expression in Aspergillus, einem filamentösen Pilz der Klasse der Ascomyceten, werden Ausbeuten bis zu 0,35 g/l in der Fermentation erzielt. Dies ist eine vergleichsweise geringe Ausbeutesteigerung im Vergleich zur Produktionsleistung von 0,1 - 0,2 g/l eines vergleichbaren Wildtypstammes in der Fermentation.

Speziell für Enzyme aus der Klasse der Basidiomyceten und hier vor allem der Weißfäulepilze besteht ein zunehmendes Interesse für industrielle Anwendungen. Als Beispiele seien genannt hydrolytische Enzyme wie Cellulasen, Hemicellulasen oder Lipasen oder aber auch Oxidoreduktasen wie Ligninperoxidasen, Manganperoxidasen, Laccasen, Cellobiose-Chinon-Oxidoreductase oder Cellobiose-Oxidase. Potentielle Anwendungen für diese Enzyme bestehen z.B. bei der Holz- bzw. Zellstoffverarbeitung.

Eine unter anderem in Basidiomyceten vorkommende Enzymklasse, die für industrielle Anwendungen von großem Interesse ist, ist die Enzymklasse der Laccasen (p-Hydroxyphenoloxidase, EC 1.10.3.2.). Laccasen gehören zur Proteinfamilie der sogenannten "blauen Kupferproteine" und enthalten in der Regel vier Kupferionen, die in drei, Typ 1 bis Typ 3 bezeichneten Kupferzentren angeordnet sind. Laccasen zeichnen sich weiter dadurch aus, daß es sich allgemein um sekretierte Proteine handelt und daß sie einen Glykosylierungsanteil von bis zu lo bis 45 % des Molekulargewichts enthalten können. Neben der Depolymerisierung von makromolekularen Verbindungen wie Lignin können Laccasen auch die Polymerisierung vor allem aromatischer Verbindungen katalysieren. Als Beispiel hierfür dient die Ligninbiosynthese in Pflanzen, bei der in Pflanzen vorkommende Laccasen beteiligt sind. Technische Anwendungsmöglichkeiten für Laccasen ergeben sich bei der Papierherstellung zur Delignifizierung von Zellstoff, bei Polymerisierungsreaktionen aller Art, z. B. bei der Abwasserbehandlung. Die Verwendung von Laccasen ist auch in der organischen chemischen Synthese bekannt, z.B. bei Kopplungsreaktionen oder der Seitenkettenoxidation von Aromaten. Für die technische Anwendung bei allen diesen Verfahren ist jedoch Voraussetzung, daß das Laccaseenzym kostengünstig und in größeren Mengen zur Verfügung gestellt werden kann.

Für verschiedene filamentöse Pilze aus der Klasse der Basidiomyceten wurden DNS-Vektoren beschrieben, die zur Transformation und Selektion von Transformanten geeignet sein sollen. Für den Basidiomyceten Phanerochaete chrysosporium wurde ein Verfahren zur homologen Transformation beschrieben (M. Alic et al. (1991) Curr. Genet. 19, 491 -494). Für den Basidiomyceten Pleurotus ostreatus wurden DNS-Konstrukte für die Transformation beschrieben (K. Yanai et al. (1996) Biosci. Biotech. Biochem. 60, 472 - 475). US 5362640 beschreibt DNS-Vektoren für die Transformation des Basidiomyceten Coriolus hirsutus. Ebenso wird für die Transformation des Basidiomyceten Coriolus versicolor ein DNS-Vektor beschrieben (Y. Iimura et al. (1992) 5th International Conference on Biotechnology in the Pulp and Paper Industry, 427 - 431). Von keinem dieser Expressionssysteme aus der Klasse der Basidiomyceten ist bekannt, daß eine signifikante Steigerung der Expressionsrate für homologe oder heterologe Proteine erreicht worden ist.

Bekannte Expressionsvektoren, die genetische Regulationselemente für die Expression in filamentösen Pilzen der Klasse der Ascomyceten enthalten, lassen sich nicht effizient in filamentösen Pilzen der Klasse der Basidiomyceten exprimieren. Sie erlauben daher bei Transformation filamentöser Pilze der Klasse der Basidiomyceten nicht die Selektion positiver Transformanten aufgrund z. B. erworbener Antibiotikaresistenz oder der Expression eines farbgebenden Indikatorpröteins oder aufgrund der Komplementation eines auxotrophen Gendefekts.

Die vorliegende Erfindung betrifft ein Expressionssystem für die Produktion eines Proteins in einem filamentösen Pilz bestehend aus
a) einem Wirtsorganismus ausgewählt aus den Gattungen Trametes und Polyporus sowie
b) einem DNS-Vektor, der ein Selektionsmarkergen enthält, welches für ein Protein kodiert das nach Transformation des Wirtsorganismus eine Selektion positiver Transformanten erlaubt und ausgewählt ist aus der Gruppe der Antibiotikaresistenzgene, die für Proteine kodieren, die die wachstumshemmende Wirkung von Antibiotika aufheben, gegen die der Wirtsorganismus nicht resistent ist, der Gene, die Proteine kodieren, die zu einer farbgebenden Reaktion befähigt sind und der Gene die einen genetischen Defekt des Wirtsorganismus (Auxotrophie) komplementieren, wobei die Expression des Selektionsmarkergens durch mindestens ein im Wirtsorganismus aktives genetisches Regulationselement kontrolliert wird und
c) einem DNS Vektor der ein Gen enthält, welches für das zu produzierende Protein kodiert wobei die Expression dieses Gens und ggf. auch die Sekretion des so produzierten Proteins durch ein im Wirtsorganismus aktives genetisches Regulationselement kontrolliert wird, wobei
   der DNS-Vektor, der ein Selektionsmarkergen enthält und der DNS-Vektor, der das Gen, welches für das zu produzierende Protein kodiert, auch als ein DNS-Vektor vorliegen können.

Als Antibiotikaresistenzgene geeignet sind vorzugsweise Gene, die Resistenz verleihen gegen ein Antibiotikum aus der Gruppe Hygromycin, Bialaphos, Kanamycin, Geneticin, Bleomycin, Oligomycin, G418, Zeocin, Benomyl und Phleomycin.

Vorzugsweise können weiter Selektionsmarkergene verwendet werden, die für Proteine kodieren, die zu einer farbgebenden Reaktion befähigt sind, z. B. das Glucuronidasegen oder das Gen für das grün fluoreszierende Protein (GFP).

Besonders geeignet sind Selektionsmarkergene, die einen genetischen Defekt des Wirtsorganismus (Auxotrophie) komplementieren können.

Für das erfindungsgemäße Expressionssystem bevorzugte Wirtsorganismen sind monokaryontische Stämme aus den Gattungen Trametes und Polyporus.

Insbesondere bevorzugt sind Wirtsorganismen der Art Trametes versicolor.

Der Wirtsorganismus im erfindungsgemäßen Expressionssystem zeichnet sich vorzugsweise dadurch aus, daß er auch einen genetischen Defekt im Metabolismus (Auxotrophie) besitzt, aufgrund dessen einer oder mehrere für das Wachstum essentielle Metaboliten nicht mehr synthetisiert werden können und der Wirtsorgansimus auf Minimalmedien ohne Zusatz dieses oder dieser Metaboliten nicht mehr zum Wachstum befähigt ist.

Die Erfindung betrifft daher auch ein Expressionssystem, welches dadurch gekennzeichnet ist, daß der Wirtsorganismus ausgewählt aus den Gattungen Trametes und Polyporus einen genetischen Defekt im Metabolismus (Auxotrophie) besitzt, aufgrund dessen einer oder mehrere für das Wachstum essentielle Metaboliten nicht mehr synthetisiert werden können und der Wirtsorganismus auf Minimalmedien ohne Zusatz dieses oder dieser Metaboliten nicht mehr zum Wachstum befähigt ist und das Selektionsmarkergen derart ausgewählt ist, daß es den auxotrophen Gendefekt des Wirtsorganismus komplementiert.

Beispiele für Selektionsmarkergene, die einen auxotrophen Gendefekt im Wirtsorganismus komplementieren können, sind das OCT-Gen (kodiert für Ornithin-Carbamoyltransferase, US 5362640), das pyr G-Gen (kodiert für Orotidin-5'-Phosphat Decarboxylase, Goosen et al., Curr. Genet. (1987) 11, 499 - 503), das trpC Gen (kodiert für ein trifunktionales Genprodukt mit Enzymaktivität für Phosphoribosyl -Anthranilsäureisomerase, Glutamin-Amidotransferase und Indol-Glycerinphosphatsynthase, Yelton et al., Proc Natl. Acad. Sci. USA (1984) 81, 1470 - 1474), oder das nia D Gen (kodiert für Nitratreduktase).

Besonders bevorzugt als Selektionsmarkergen ist das pyr G Gen, das für die Orotidin-5'-Phosphat Decarboxylase (ein Enzym des Uridinstoffwechsels) kodiert, und die Uridin Auxotrophie eines im pyrG Gen defekten Wirtsstammes komplementieren kann. Als Wirtsorganismus besonders bevorzugt ist dabei ein Stamm aus der Gattung Trametes oder Polyporus mit einem Defekt im pyr G Gen der für Uridin auxotroph ist.

Das pyr G Gen stammt bevorzugt aus einem Pilz aus der Klasse der Basidiomyceten, z.B. aus der Gattung Agaricus, Coriolus, Polyporus, Pleurotus, Phanerochaete, Schizophyllum oder Trametes.

Bevorzugt geeignet für die Expression des pyr G Gens sind die Promotor- und Terminatorelemente für ein Glycerinaldehyd-3-Phosphat Dehydrogenasegen (GAPDH Gen) beispielsweise aus den filamentösen Pilzen der Klasse der Basidiomyceten, Schizophyllum commune, Agaricus bisporus (das GAPDH Ag2 Gen), Phanerochaete chrysosporium, Trametes versicolor oder fur ein Laccase Gen, vorzugsweise das Laccase I Gen, bzw. das Laccase III Gen aus Trametes versicolor.

Insbesonders geeignet als Selektionsmarkergen für das erfindungsgemäße Expressionssystem sind das Orotidin-5'-Phosphat Decarboxylasegen (pyr G Gen) vorzugsweise aus dem Basidiomyceten Schizophyllum commune oder aus dem Basidiomyceten Trametes versicolor.

Vorzugsweise wird die Expression des pyr G Gens aus dem Basidiomyceten Schizophyllum commune vom Promotor und ggf. Terminator für das pyr G Gen aus Schizophyllum commune reguliert.

Vorzugsweise wird die Expression des pyr G Gen aus dem Basidiomyceten Trametes versicolor, vom Promotor für das pyr G Gen aus Trametes versicolor reguliert.

Das erfindungsgemäße Expressionssystem eignet sich insbesondere zur Expression eines Gens das für ein hydrolytisches Enzym z.B. aus der Gruppe der Cellulasen, Hemicellulasen und Lipasen oder aus der Gruppe der Oxidoreduktasen wie z. B. den Ligninperoxidasen, Manganperoxidasen, Laccasen, Cellobiose-Chinon-Oxidoreductase oder Cellobiose-Oxidase kodiert.

Insbesondere bevorzugt eignet es sich zur Expression eines Gens für eine Laccase.

Die Erfindung betrifft auch Verfahren zur Herstellung von Pilzstämmen, die zur effizienten Expression und Sekretion von Proteinen befähigt sind.

Dieses Verfahren ist dadurch gekennzeichnet, daß als Wirtsorganismus ein Pilz ausgewählt aus den Gattungen Trametes und Polyporus mit einem auxotrophen Gendefekt verwendet wird, welcher mittels an sich bekannter Verfahren mit einem DNS-Vektor, der ein Gen zur Komplementation des auxotrophen Gendefekts im Wirtsstamm besitzt, transformiert wird und aus dem Transformationsansatz die mit dem DNS-Vektor transformierten Klone durch Selektion auf Komplementation des auxotrophen Gendefekts ausgewählt werden, wobei die Expression des Gens zur Komplementation des auxotrophen Gendefekts im Wirtsstamm durch ein genetisches Regulationselement kontrolliert wird, das im Wirtsstamm aktiv ist.

Filamentöse Pilze, die als Wirt für die Genexpression verwendet werden können, gehören zur Gattung Trametes oder Polyporus.

Bevorzugt als Wirt für die Genexpression ist ein monokaryontischer Basidiomycet aus der Gattung Trametes, oder Polyporus.

Weiterhin ist es von Vorteil, wenn die genannten Wirtsstämme aus der Klasse der Basidiomyceten einen auxotrophen Gendefekt haben, der als Selektionsmarker zur Identifizierung positiver Transformanten verwendet werden kann. Verwendet werden können beispielsweise Wirtsstämme aus der Klasse der Basidiomyceten, die einen Gendefekt im OCT-Gen, im pyr G-Gen, im trpC Gen oder im nia D Gen haben.

Bevorzugt als Wirt für die Genexpression ist ein Pilz ausgewählt aus den Gattungen Trametes und Polyporus der einen Defekt im pyr G-Gen hat.

Insbesondere bevorzugt für die Genexpression ist ein Wirt der Art Trametes versicolor, der einen Defekt im pyr G-Gen hat und für Uridin auxotroph ist.

Die Transformation des Wirtsstammes erfolgt nach Methoden, die dem Stand der Technik entsprechen. Zu diesen Methoden gehören die Transformation von Protoplasten nach der CaCl₂/PEG-Methode, die Transformation durch Elektroporation oder die biolistische Transformation durch Beschuß mit DNShaltigen Mikroprojektilen. Diese Verfahren sind in Standardlehrbüchern beschrieben.

Beispielsweise wird das zu transformierende Gen in bekannter Art und Weise in einen DNS-Vektor der mindestens ein Selektionsmarkergen enthält, welches für ein Protein kodiert das nach Transformation eines Pilzes ausgewählt aus den Gattungen Trametes und Polyporus eine Selektion positiver Transformanten erlaubt, wobei das Selektionsmarkergen ausgewählt ist aus der Gruppe der Antibiotikaresistenzgene, die für Proteine kodieren, die die wachstumshemmende Wirkung von Antibiotika aufheben, gegen die der Wirtsorganismus nicht resistent ist, der Gene, die Proteine kodieren, die zu einer farbgebenden Reaktion befähigt sind und der Gene die einen genetischen Defekt des Wirtsorganismus (Auxotrophie) komplementieren und wobei das Selektionsmarkergen durch mindestens ein im Wirtsorganismus aktives genetisches Regulationselement kontrolliert wird, kloniert und mittels der genannten Methoden in einen filamentösen Pilz ausgewählt aus den Gattungen Trametes und Polyporus eingebracht.

Das zu transformierende Gen kann aber auch in einen Expressionsvektor ohne Selektionsmarkergen kloniert werden und zusammen mit einem den auxotrophen Gendefekt des Wirtsstammes komplementierenden Vektor zur Erzeugung von Transformanten verwendet werden (Cotransformation).

Bevorzugter Stamm für die Transformation ist ein filamentöser Pilz ausgewählt aus den Gattungen Trametes und Polyporus . Bei dem betreffenden Stamm aus der Klasse der Basidiomyceten kann es sich dabei um einen monokaryontischen oder aber auch um einen dikaryontischen Stamm handeln. In einer bevorzugten Ausführung handelt es sich um einen Uridin-auxotrophen Stamm, der einen Defekt im pyr G-Gen hat.

Besonders bevorzugt für die Transformation ist ein monokaryontischer, Uridin-auxotropher, pyr G defizienter Stamm aus der Art Trametes versicolor.

Die Selektion positiver Transformanten erfolgt beispielsweise, indem Protoplasten nach der Transformation mit Vektor DNS auf einem Medium ausgebracht werden, welchem zur osmotischen Stabilisierung der Protoplasten ein Zusatz wie z. B. Sorbitol, Mannitol oder Saccharose beigefügt ist und welches die Selektion von Transformanten mit dem komplementierenden pyr G-Gen erlaubt.

In einer bevorzugten Ausführung der Erfindung wird in einem homologen System der filamentöse Pilz Trametes versicolor mit dem Gen einer Laccase aus Trametes versicolor transformiert Dadurch wird eine Steigerung der Expressionsrate für die besagte Laccase erzielt, die die nach dem Stand der Technik erzielbare Produktionsrate in der Fermentation von 0,1 g Laccase/l Kulturmedium signifikant verbessert.

Vorzugsweise verwendet man dazu den Promotor der dem Laccasegen eigen ist oder den Promotor für ein stark exprimiertes Gen aus Trametes versicolor. Vorzugsweise verwendet man die Promotoren der Laccasegene I und III, deren Isolierung im 4.

Beispiel beschrieben wird. Von dem Laccase I Gen wird dabei vorzugsweise der in SEQ ID NO: 1 enthaltene Sequenzabschnitt von Base 1 - 1192 verwendet. Von dem Laccase III Gen wird vorzugsweise der in SEQ ID NO: 2 enthaltene Sequenzabschnitt von Base 1 - 547 verwendet. Den Promotor eines weiteren stark exprimierten Gens stellt der GAPDH Promotor für die Glycerinaldehyd-3-Phosphatdehydrogenase aus Trametes versicolor dar. Die Isolierung des GAPDH-Promotors wird im 5. Beispiel beschrieben. Die GAPDH-Promotorsequenz entspricht dem in SEQ ID NO: 3, Base 1 - 1542 aufgeführten Sequenzabschnitt. Es zeigte sich, dass insbesondere SEQ ID NO: 3, Base 1365 bis bp 1542 sowie zu diesem Sequenzabschnitt homologe Sequenzen mit einer Homologie größer als 73 % für die Expression geeignet sind. Vorzugsweise ist ferner noch mindestens einer der folgenden Sequenzabschnitte ebenfalls auf dem erfindungsgemäßen Regulationselement vorhanden:
SEQ ID NO: 3: Base 1005 bis bp 1123 sowie zu diesem Sequenzabschnitt homologe Sequenzen mit einer Homologie größer als 52 %, oder
SEQ ID NO: 3: Base 817 bis bp 947 sowie zu diesem Sequenzabschnitt homologe Sequenzen mit einer Homologie größer als 44 %, oder
SEQ ID NO: 3: Base 640 bis bp 728 sowie' zu diesem Sequenzabschnitt homologe Sequenzen mit einer Homologie größer als 50 %.

In der vorliegenden Erfindung beziehen sich alle erwähnten Homologiewerte auf Ergebnisse, die mit dem Computerprogramm "Wisconsin Package Version 9.1, Genetics Computer Group (GCG), Madison, Wisconsin" erhalten werden. Die Homologiebestimmung erfolgt durch eine Suche in der Datenbank mit dem Unterprogramm "fasta" und den voreingestellten Werten (word size 6). Die ähnlichsten Sequenzen werden dann mit dem Unterprogramm "gap" auf Homologie untersucht. Hierbei werden die voreingestellten Parameter "gap creation penalty 50" und "gap extension penalty 3" verwendet. Zusätzlich wurde mit dem Unterprogramm "gap" und den oben genannten voreingestellten Parametern die in JP 09047289 offenbarte, aber noch nicht in einer Datenbank verfügbare Promotorsequenz des GAPDH-Gens aus Coriolus hirsutus auf Homologie untersucht.

Die Erfindung betrifft somit auch ein in Trametes oder Polyporus aktives Regulationselement, welches dadurch gekennzeichnet ist, daß es den in SEQ ID NO: 1 enthaltenen Sequenzabschnitt von Base 1 - 1192 oder den in SEQ ID NO: 2 enthaltenen Sequenzabschnitt von Base 1 - 547 oder den in SEQ ID NO: 3, enthaltenen Sequenzabschnitt von Base 1365 - 1542 sowie zu diesem Sequenzabschnitt homologe Sequenzen mit einer Homologie größer als 73 % umfaßt.

Vorzugsweise verwendet man Selektionsmedien, auf denen nur solche Transformanten von Trametes versicolor wachsen können, die mit einem funktionell exprimierten Selektionsmarkergen für das pyr G-Gen transformiert worden waren. Bevorzugt handelt es sich um ein im 2. Beispiel beschriebenes Minimalmedium in Abwesenheit von Uridin, auf dem pyr G auxotrophe Stämme von Trametes versicolor nicht mehr wachsen können, bzw. erst nach Zusatz von Uridin wieder wachsen können.

Die erfolgreiche Anwendung eines DNS-Vektors enthaltend das pyr G Gens als Selektionssystem ist abhängig von der effizienten Expression des Selektionsmarkergens in Trametes Transformanten. Für eine effiziente Expression sind entsprechende Expressionssignale notwendig.

In Trametes versicolor bewirken Expressionssignale aus Basidiomyceten mit überraschenderweise erheblich höherer Effizienz eine funktionelle Expression als die anderweitig verfügbaren Expressionssignale aus Ascomyceten. Deshalb stehen bei den erfindungsgemäßen DNS Vektoren das pyr G Selektionsmarkergen vorzugsweise unter der Kontrolle von genetischen Regulationselementen aus Basidiomyceten, insbesondere bevorzugt von solchen ausgewählt aus den Gattungen Trametes und Polyporus .

Vorzugsweise steht das pyr G-Gen unter der Kontrolle der ihm vorgeschalteten 5'-Promotorregion sowie der ihm nachgeschalteten 3'-Terminatorregion. Ein DNS-Fragment, bei dem das pyr G-Gen aus Schizophyllum commune unter Kontrolle der Expressionssignale des pyr G-Gens aus Schizophyllum commune steht, ist von Froeliger et al., Gene (1989) 83, 387 - 393 beschrieben. Weiterhin kann das pyr G-Gen unter der Kontrolle von Expressionssignalen aus Basidiomyceten stehen, die verschieden von denen des pyr G-Gens sind. Zu den Expressionssignalen, die diese Funktion erfüllen, gehören GAPDH-Promotoren filamentöser Pilze aus der Klasse der Basidiomyceten, wie z.B. Coriolus hirsutus, Phanerochaete chrysosporium, Agaricus bisporus oder Trametes versicolor, der OCT-Promotor aus Coriolus hirsutus, der Promotor der Laccase I oder der Laccase III aus Trametes versicolor sowie der Terminator des GAPDH-Gens aus Agaricus bisporus oder die Terminatoren des Laccase I, bzw. Laccase III Gens aus Trametes versicolor.

Besonders bevorzugt ist ein Vektor, der im 3. Beispiel beschrieben wird, bei dem das pyr G-Gen aus Schizophyllum commune unter Kontrolle der Expressionssignale des pyr G-Gens aus Schizophyllum commune steht.

Das pyr G-Gen kann jedes Gen sein, das für ein Protein mit der enzymatischen Aktivität einer Orotidin-5'-Phosphatdecarboxylase kodiert. Bevorzugt stammt das pyr G-Gen aus einem filamentösen Pilz aus der Klasse der Basidiomyceten, wie z.B. Agaricus bisporus, Phanerochaete chrysosporium, Coriolus hirsutus, Polyporus pinsitus, Schizophyllum commune oder Trametes versicolor.

Besonders bevorzugt ist das pyr G-Gen aus Schizophyllum commune und Trametes versicolor.

Bei den exprimierten und sekretierten Proteinen kann es sich um heterologe Proteine oder aber um homologe Proteine handeln.

Vorzugsweise handelt es sich um eine Laccase. Solche Laccasen sind beispielsweise aus dem Stamm Trametes versicolor bekannt (Beispiele für Laccasegene sind das Gen für Laccase I: SEQ ID NO: 1, Base 1193 - 3312, oder das Gen für Laccase III: SEQ ID NO: 2, Base 548 - 2542).

Die Erfindung betrifft somit auch ein Verfahren zur Herstellung von Proteinen, welches dadurch gekennzeichnet ist, daß das erfindungsgemäße Expressionssystem in an sich bekannten Weise zur Proteinproduktion eingesetzt wird oder daß ein Pilzstamm der nach dem erfindungsgemäßen Verfahren hergestellt wurde in an sich bekannter Art und Weise kultiviert wird.

Solche Herstellungsverfahren sind beispielsweise bekannt aus CA: AN 96-203142, Eggert et al., Appl. Environ. Microbiol. (1996) 62, 1151 - 1158, Martinez et al., Appl. Microbiol. Biotechnol. (1994) 41, 500 - 504, oder WO 93/08272.

Die folgenden Beispiele dienen der weiteren Erläuterung der Erfindung. Die in den Beispielen verwendeten Standardmethoden zur Behandlung von DNS oder RNS, wie die Behandlung mit Restriktionsendonukleasen, DNS Polymerasen, Reverser Transkriptase etc. sowie die Standardverfahren wie Transformation von Bakterien, Southern und Northern Analyse, DNS Sequenzierung, radioaktive Markierung, Screening und PCR-Technologie wurden, wenn nicht anders angegeben, durchgeführt wie vom Hersteller empfohlen oder wenn keine Herstelleranleitung vorhanden war entsprechend dem aus den Standardlehrbüchern bekannten Stand der Technik.

### 1. Beispiel:

### Herstellung von Trametes Protoplasten und Regenerierung von Pilzkolonien

Für die Gewinnung von Protoplasten verwendet wurden die dikaryotischen Stämme Trametes versicolor TV-1 (hinterlegt bei der DSMZ Deutschen Sammlung von Mikroorganismen und Zellkulturen GmbH, D-38124 Braunschweig unter der Nummer DSM 11523), Trametes versicolor 38070 (erhältlich von American Type Culture Collection, Rockville, MD 20852 USA) und der monokaryotische Stamm Trametes versicolor F2 100 (hinterlegt bei der DSMZ Deutschen Sammlung von Mikroorganismen und Zellkulturen GmbH, D-38124 Braunschweig unter der Nummer DSM 11972). Mycel von Trametes versicolor wurde zuerst durch Anzucht für 7 Tage bei 28°C auf Malzagarplatten (3 % Malz Extrakt, 0,3 % Pepton aus Sojabohnenmehl, 1,5 % Agar-Agar, pH 5,0) gewonnen. Von den Malzagarplatten wurden drei Stücke ausgestanzt und damit 100 ml steriles 1.5 % Malzextrakt Medium (3 % Malz Extrakt, 0,3 % Pepton aus Sojabohnenmehl, pH 5,0) in 500 ml Erlenmeyerkolben, bzw. 125 ml des sterilen Mediums in 162 cm² Zellkulturgefäßen, angeimpft. Die Kultur wurde ohne Schütteln für 7 Tage bei 28°C inkubiert, bis die Kulturflüssigkeit dicht mit einer Mycelmatte bewachsen war. Die Kulturflüssigkeit wurde abdekantiert und frisches Medium zugegeben (30 ml für das Mycel einer 100 ml Anzucht). Das Mycel wurde mit einem Ultra Turrax (9500 rpm, 4 min) homogenisiert und unter Schütteln bei 100 rpm für weitere 18 h bei 28°C inkubiert.

Die auf diese Weise hergestellte Mycelsuspension wurde durch Zentrifugation für 5 min bei 1500 rpm (2000 x g) geerntet und das dabei erhaltene Mycel dreimal durch suspendieren mit 0.1 M MgSO₄, 0,6 M Saccharose, 0,1 M Phosphat, pH 5,8 (OMT-Medium) und anschließendes Zentrifugieren gewaschen. Das isolierte Mycel wurde gewogen und bis, zur Behandlung mit lytischem Enzym bei 4°C aufbewahrt.

Protoplasten wurden folgendermaßen hergestellt: In einem sterilen Erlenmeyerkolben wurde Mycelium eines Kolbens in 15 ml einer frisch hergestellten und sterilfiltrierten Lösung des lytischen Enzymgemisches Novozym 234 (3 mg/ml, Novo Nordisk, Bagsvaerd, Dänemark) in OMT-Medium suspendiert. Das in der Enzymlösung resuspendierte Mycelium wurde bei geringer Drehzahl (80 rpm) auf einem Schüttelinkubator (Infors) für 1 bis 3 h bei 30°C inkubiert. Während der Inkubation wurde die Bildung der Protoplasten im Mikroskop beobachtet. Frei bewegliche Protoplasten waren üblicherweise nach 1 h zu sehen. Der Endpunkt der Protoplastierung wurde durch visuelle Inspektion im Mikroskop bestimmt und die Protoplasten durch Filtration über Glaswolle in einem Glasfilter von restlichem Mycelium abgetrennt. Die Glaswolle wurde sorgfältig mit eisgekühltem OMT-Medium gewaschen. Protoplasten wurden durch Zentrifugation der Suspension in einem sterilen Probengefäß isoliert (2000 rpm; 2500 x g, 4°C, 10 min). Die weitere Bearbeitung der Zellen erfolgte bei 4°C. Das Protoplastenpellet wurde durch suspendieren in OMT-Medium gewaschen und durch Zentrifugation reisoliert. Bei Bedarf wurde der Waschschritt wiederholt. Die Konzentration von Protoplasten wurde unter dem Mikroskop in einer Zählkammer bestimmt. Die Protoplastensuspension wurde für Experimente zur Protoplastenregenerierung oder für Transformationen auf eine Konzentration von 1 x 10⁸ Protoplasten/ml eingestellt.

Für Regenerierungsexperimente würden von der Protoplastensuspension Verdünnungsreihen hergestellt und auf Agarplatten ausplattiert, die 1.5 % Malzextrakt, 0.1 % Trypticase Pepton, 2 % Glucose, 1.5 % Agar und zur osmotischen Stabilisierung 0.4 M Mannitol enthielten. Auf diese Weise wurde der Anteil an überlebensfähigen Zellen bestimmt und getestet, ob die erhaltenen Protoplasten zu mycelartigem Wachstum regeneriert werden konnten. Auf die gleiche Weise wurde auf osmotisch nicht stabilisierten Platten (ohne Mannitol) der Anteil an osmotisch stabilen Zellen (z.B. Mycelfragmente) bestimmt. Nach Inkubation bei 28°C für 7 Tage wurden die erhaltenen Kolonien gezählt. Der Anteil überlebensfähiger Zellen aus einer Reihe von Protoplastenpräparationen betrug ca. 0.5 %. Diese Ergebnisse zeigen, daß von Trametes versicolor überlebensfähige und regenerierbare Protoplasten für Transformationsexperimente hergestellt werden können.

### 2. Beispiel

### Isolierung von pyr G-defizienten Mutanten von Trametes versicolor

Auxotrophe Mutanten von Trametes versicolor mit einem Gendefekt im Pyrimidin Stoffwechsel (pyr-Mutanten) wurden in Anlehnung des in Boeke et al., Methods Enzymol. (1987) 154, 164 - 175 beschriebenen Verfahrens isoliert. Als selektives Agens wurde die genotoxische Substanz 5-Fluor-Orotinsäure (FOA) verwendet. Mutagenese von Trametes versicolor Protoplasten erfolgte durch UV-Behandlung.

### A: UV-Mutagenese:

Für die Mutagenese verwendet wurde der im 1. Beispiel beschriebene monokaryontische Stamm Trametes versicolor F2 100. Protoplasten dieses Stammes wurden hergestellt wie im 1. Beispiel beschrieben.

Für die Mutagenese wurde ein BioRad UV-Linker (5,8 W/cm², Abstand von der UV-Quelle 16 cm) verwendet. Die Zahl der für die Mutagenese verwendeten Protoplasten betrug 8 x 10⁹. Protoplasten von Trametes versicolor wurden in einer Petrischale vorgelegt und für verschieden lange Zeitabschnitte mit UV-Licht bestrahlt. Dabei stellte sich heraus, daß unter den beschriebenen Bedingungen eine Bestrahlung für 60 sec. optimal für die nachfolgende Selektion auxotropher Mutanten war.

### B: Selektion von Uridin auxotrophen Mutanten:

Für die Selektion von Uridin auxotrophen Mutanten wurde folgendes Minimalmedium (MM) verwendet:

| | |
|---|---|
| Glucose | 20 g/l |
| Agar | 15 g/l |
| Kaliumdihydrogenphosphat | 1 g/l |
| Magnesiumsulfat | 0,5 g/l |
| Dinatriumhydrogenphosphat | 0,1 g/l |
| Adenin | 27,5 mg/l |
| DL-Phenylalanin | 0,15 g/l |
| L-Asparagin | 2,5 g/l |
| Thiamin | 0,48 mg/l |
| Calciumchlorid | 10 mg/l |
| Eisensulfat | 10 mg/l |
| Kupfersulfat | 2 mg/l |
| Zinksulfat | 1 mg/l |
| Mangansulfat | 1 mg/l |
| pH 5,0, mit Schwefelsäure eingestellt. | |

Für die osmotische Stabilisierung von Protoplasten wurde das MM mit 0.6 M Saccharose supplementiert (MMS). Für Flüssiganzuchten wurde das MM ohne Agar hergestellt.

Zu Beginn wurde das MMS mit verschiedenen Konzentrationen FOA sowie 10 mM Uridin supplementiert, um die Wachstumseigenschaften auf selektivem Medium für verschiedene Trametesstämme zu charakterisieren. Es stellte sich heraus, daß MMS mit 1,5 mg/ml FOA und 10 mM Uridin (selektives MMS) das Wachstum der untersuchten Trametesstämme vollständig unterdrückte.

Platten mit selektivem MMS wurden mit UV-mutagenisierten Protoplasten (in Abschnitt A beschrieben) beimpft und 21 Tage bei 28°C inkubiert. Im Gegensatz zu nicht mutagenisierten Protoplasten wurde das Wachstum von 35 Kolonien beobachtet. Diese potentiellen pyr-defizienten Mutanten wurden, um den Uridin auxotrophen Phänotyp näher zu charakterisieren, auf MM-Platten, MM-Platten mit 10 mM Uridin und selektiven MM-Platten ausgebracht und das Wachstum zum Ausgangsstamm F2 100 verglichen. Dabei zeigten von den 35 gepickten Kolonien drei Trametesmutanten eindeutig einen pyr-defizienten Phänotyp. Dies ist in der Tabelle 1 dargestellt.

**Tabelle 1**

| Wachstum von Trametes versicolor Mutanten auf verschiedenen Minimalmedien | | | |
|---|---|---|---|
| Stamm | MM | MM + 10 mM Uridin | MM + 10 mM Uridin + 1,5 mg/ml FOA |
| F2 100 | + | + | - |
| F2 100B11 | - | + | + |
| F2 100B14 | - | + | + |
| F2 100B16 | - | + | + |

### C: Identifizierung von pyr G-Mutanten

Die Mutagenese mit FOA kann entweder zu Mutanten im pyr F Gen (Orotsäure-Phosphoribosyltransferase) oder im gewünschten pyr G Gen (Orotidin-5'-Phosphatdecarboxylase) führen, pyr G Mutanten und pyr F Mutanten wurden differenziert durch Transformation mit dem pyr G Gen aus Schizophyllum commune (Plasmid pSCpyrG siehe 3. Beispiel).

Von den drei in Tabelle 1 aufgeführten pyr-defizienten Mutanten konnten nach Transformation mit dem Plasmid pSCpyrG Kolonien auf MM beobachtet werden. Dies deutet darauf hin, daß alle drei Mutanten defizient im pyr G Gen waren. Der pyr G-defiziente Trametes versicolor Stamm F2 100B11 ließ sich jedoch mit einer ca. 10-fach höheren Häufigkeit transformieren, so daß dieser Stamm für die weiteren Untersuchungen verwendet wurde.

Bei den Stämmen Trametes versicolor F2 100B11, F2 100B14 und F2 100B16 handelt es sich um die ersten bisher beschriebenen pyr G-defizienten Stämme von Trametes versicolor. Diese pyr G-defizienten Stämme können als Wirtsorganismen dienen für die bisher nicht beschriebene Transformation von Trametes versicolor und sind somit neue wertvolle Wirtsorganismen für die Proteinexpression und Proteinsekretion in filamentösen Pilzen aus der Klasse der Basidiomyceten. Die Verwendung des Stammes F2 100B11 zu diesem Zweck wird in den folgenden Beispielen beschrieben.

### 3. Beispiel

### Transformation von pyr G auxotrophen Trametes versicolor Stämmen mit dem pyr G-Gen aus Schizophyllum commune

### A: Isolierung des pyr G Gens aus Schizophyllum commune

Die Isolierung und DNS-Sequenz des pyr G Gens aus Schizophyllum commune (in der Publikation bezeichnet als URA1 Gen) ist beschrieben in Froeliger et al., Gene (1989) 83, 387 - 393. Mycelium von Schizophyllum commune (Stamm ATCC 44201, bezogen von der ATCC American Type Culture Collection, 12301 Parklawn Drive, Rockville, Maryland 20852-1776, USA) wurde in Malzextraktmedium hergestellt wie im 1. Beispiel für Trametes versicolor beschrieben. Die Isolierung von chromosomaler DNS folgte ebenfalls der Methode für Trametes versicolor und wird im 4. Beispiel beschrieben. Das pyr G Gen wurde aus chromosomaler DNS von Schizophyllum commune durch PCR amplifiziert. Dazu wurden die Primer A und B verwendet (abgeleitet von der publizierten Sequenz in Froeliger et al., Gene (1989) 83, 387 - 393), mit denen neben dem kodierenden Bereich auch Promotorund Terminatorsequenzen amplifiziert werden konnten.

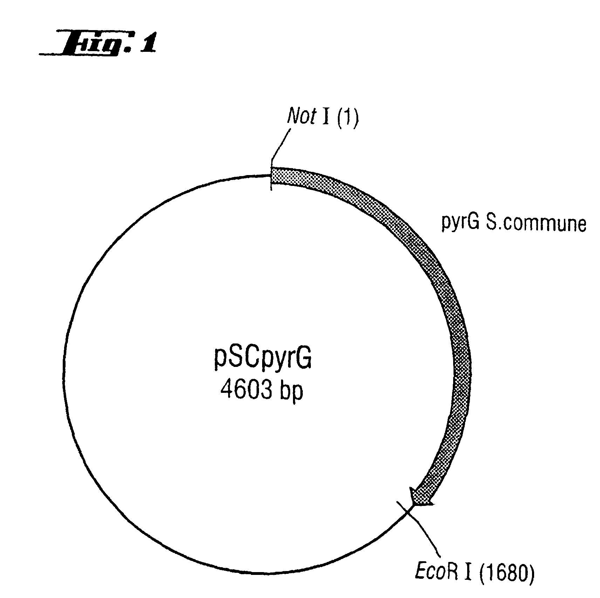

PCR-Amplifikationen wurden entsprechend dem Stand der Technik nach den Angaben des Herstellers (PCR Kit von Stratagene) durchgeführt: Es wurden 200 ng chromosomaler S. commune DNS in einer 100 µl PCR eingesetzt, die den vom Hersteller bereitgestellten Puffer enthielt und darüberhinaus 1,25 U Pfu Polymerase, je 0,2 mM der vier dNTPs (dATP, dCTP, cGTP, dTTP) und jeweils 100 pmol der Primer A und B. Die weiteren Bedingungen zur spezifischen Amplifikation des gewünschten PCR-Produkts waren: 5 min bei 94°C, gefolgt von 25 Zyklen von 1 min bei 94°C, 1.5 min bei 50°C und 2.5 min bei 72°C sowie abschließend 5 min bei 72°C. Das gewünschte PCR-Produkt von ca. 1.6 kb Größe wurde durch Agarose Gelelektrophorese gereinigt und in den PCR-Script SK(+) Vektor (Stratagene) kloniert und in E. coli transformiert. Aus der Anzucht transformierter E. coli wurde das Plasmid isoliert. Von einem positiven Klon wurde durch DNS-Sequenzanalyse des Inserts bestätigt, daß das S. commune pyr G Gen amplifiziert worden war. Das Plasmid mit dem S. commune pyr G Gen wurde pSCpyrG (Fig. 1) bezeichnet.

### B: Transformation von Trametes versicolor pyr G defizienten Stämmen

Protoplasten von T. versicolor F2 100B11 (siehe 2. Beispiel) wurden nach dem im 1. Beispiel beschriebenen Verfahren hergestellt. Dabei war das Anzuchtsmedium für den auxotrophen Stamm mit 10 mM Uridin supplementiert worden. Es wurde mit dem Vektor pSCpyrG (beschrieben in Abschnitt A) transformiert.

Wie im 1. Beispiel beschrieben, wurden Protoplasten von Trametes versicolor F2 100B11 hergestellt und in einer Endkonzentration von 10⁸/ml suspendiert. In Inkubationsgefäßen von 12 ml Volumen wurden 0.1 ml Aliquots der Protoplasten mit je 10 µg DNS des Plasmids gemischt und 30 min auf Eis inkubiert. Danach wurde langsam und unter wiederholtem mischen 1,25 ml einer PEG4000 Lösung zum Transformationsansatz gegeben. Nach weiteren 20 min Inkubation bei Raumtemperatur wurden die Reaktionsgefäße mit dem im 1. Beispiel beschriebenen OMT-Medium aufgefüllt, gemischt und 10 min bei 4°C bei 2000 x g zentrifugiert. Die Pellets wurden resuspendiert und auf osmotisch stabilisierten MMS-Platten ohne Uridin (beschrieben im 2. Beispiel) ausplattiert. Die Platten wurden bei 28°C für 14 Tage inkubiert und auf Wachstum von Kolonien überprüft. Bei verschiedenen Experimenten wurden Transformationsraten von 0,1 - 3 Transformanten/µg Plasmid DNS erzielt.

### C: Reinigung der Transformanten

Mycel der erhaltenen Transformanten wurde gepickt und durch ausplattieren auf frische Platten mit MM gereinigt. Dabei wurde das Inokulum punktförmig in der Mitte der Platte aufgebracht. Nach Inkubation für 7 bis 14 Tagen bei 28°C konnte radiales Mycelwachstum beobachtet werden. Dieser Reinigungsvorgang wurde wiederholt, wobei das Mycel für das Inokulum vom Rand der ersten Reinigungsplatte entnommen wurde. MM-Platten wurden dann erneut mit Inokulum der zweiten Reinigungsplatte inokuliert und bei 28°C inkubiert, bis die Platten vollständig mit Mycel bewachsen waren.

### D: Analyse der Transformanten

Transformanten von Trametes versicolor wurden mittels Southernblot Analyse auf die Integration des Plasmids pSCpyrG untersucht. Dazu wurde von verschiedenen Transformanten und als Kontrolle dem pyr G defizienten Stamm F2 100B11 Mycel in Flüssigkultur hergestellt (siehe 1. Beispiel, Malzextraktmedium, mit 10 mM Uridin versetzt). Von dem isolierten Mycel wurde chromosomale DNS isoliert wie nachfolgend im 4. Beispiel beschrieben.

Von den untersuchten Stämmen wurden je 1 µg der chromosomalen DNS und 100 ng des Plasmids pSCpyrG durch einen Doppelverdau mit Not I und Eco RI geschnitten, anschließend durch Agarose Gelelektrophorese getrennt, auf Nylonfilter (Qiagen) geblottet und mit einer radioaktiv markierter DNS-Sonde hybridisiert, die spezifisch für das pyr G Gen aus Schizophyllum commune war.

Die DNS-Sonde wurde vorbereitet, indem das Plasmid pSCpyrG durch einen Doppelverdau mit Not I und Eco RI geschnitten und das dabei entstandene 1,4 kb lange DNS-Fragment des pyr G Gens durch präparative Gelelektrophorese isoliert wurde. Das 1,6 kb lange pyr G-spezifische DNS-Fragment wurde mit α-[³²P]-dATP radioaktiv markiert ("Random Priming" Kit, Boehringer Mannheim). Freie Radioaktivität wurde durch Chromatographie über Sephadex G25 (Pharmacia) abgetrennt. Die spezifische Aktivität der radioaktiv markierten DNS-Sonde betrug 1 x 10⁷ cpm/µg DNS. Die Hybridisierungstemperatur für die auf Nylonfilter geblottete DNS mit der radioaktiv markierten DNS-Sonde betrug 60°C. Ansonsten wurden die in der Fachliteratur beschriebenen Bedingungen für Southernblots eingehalten. Southernblots wurden durch Autoradiographie ausgewertet. Dabei konnte das 1,6 kb lange pyr G-spezifische DNS-Fragment nur in den Transformanten, nicht jedoch in dem Uridin-auxotrophen Stamm F2 100B11 nachgewiesen werden. Dieses Ergebnis bestätigt, daß bei der Transformation des Uridin-auxotrophen Stammes Trametes versicolor F2 100B11 das Plasmid pSCpyrG in das Genom integriert worden war und zur produktiven Expression des Selektionsmarkergens pyr G führte, wodurch die Uridin Auxotrophie dieses Stammes komplementiert wurde. Überraschenderweise wurde dabei auch erstmals festgestellt, daß die Expressionssignale des pyr G Gens aus dem Basidiomyceten Schizophyllum commune auch in Trametes versicolor funktionsfähig sind.

### 4. Beispiel

### Klonierung von T. versicolor Laccase Genen

### A: Herstellung einer chromosomalen Genbank aus Trametes versicolor.

Genomische DNS von *T. versicolor* wurde aus dem Mycel einer Schüttelkolbenkultur isoliert. 1 g Mycel von *T. versicolor* wurden in Gegenwart von flüssigem Stickstoff mit Mörser und Pistill zu einem feinen Pulver zerrieben. Das Pulver wurde in einem sterilen Probengefäß aufgenommen und sofort mit 5 ml Extraktionslösung (0.1 M Tris-HCl, pH 8.0, 0.1 M EDTA, 0.25 M NaCl, 0.6 mg/ml Proteinase K) und 0.5 ml einer 10 % (w/v) Natriumlauroylsarcosinlösung versetzt. Nach Incubation bei 50°C für mindestens 2 h wird das Gemisch mit 0.85 ml 5 M NaCl und 0.7 ml einer 10 % (w/v) CTAB-Lösung in 0.7 M NaCl versetzt und 30 min bei 65°C inkubiert. Nach Zugabe von 7 ml eines Chloroform-Isoamylalkoholgemisches (24:1) wird der Ansatz geschüttelt und die beiden Phasen durch Zentrifugation getrennt. Die wässrige Phase wird entfernt und chromosomale DNS durch Zugabe von 0.6 Volumenanteilen Isopropanol gefällt. Die gefällte DNS wird anschließend über eine Säule (Qiagen Genomic Tip) gereinigt. Auf diese Weise konnten aus 16 g Mycel 0,5 mg chromosomale DNS isoliert werden.

Zur Herstellung der chromosomalen Genbank wurden 90 µg chromosomale DNS von Trametes versicolor TV-1 in einem Partialverdau mit Sau 3A geschnitten und durch Agarose Gelelektrophorese aufgetrennt. Die chromosomalen DNS-Fragmente wurden im Größenbereich von 5 - 20 kb und größer 20 kb isoliert und jeweils in mit Bam HI vorgeschnittene Lambda-Phagen kloniert (Stratagene Klonierungssystem). Von der 5- 20 kb DNS-Fraktion wurden 4 x 10⁴ Phagen/µg Vektor-DNS und von der DNS-Fraktion größer 20 kb wurden 5 x 10⁴ Phagen/µg Vektor-DNS erhalten. Die Phagen wurden durch Infektion des E. coli Stammes XL-1 Blue MRF' amplifiziert.

### B: Isolierung einer Laccase-spezifischen DNS Sonde

Eine DNS-Sonde zur Isolierung von Laccasegenen wurde durch PCR-Amplifikation aus *T. versicolor* genomischer DNS mit degenerierten Primern erzeugt. Die degenerierten Primer wurden anhand eines Sequenzvergleichs bekannter Laccasegene konstruiert. Es wurden die Aminosäuresequenzen der in der EMBL Gendatenbank enthaltenen Laccasegene von Neurospora crassa, Coriolus hirsutus, Phlebia radiata, Agaricus bisporus und eines nicht näher charakterisierten filamentösen Pilzes aus der Unterklasse der Basidiomyceten verglichen. Durch den Sequenzvergleich konnten vier Peptide mit einer Länge von 5 bis 7 Aminosäuren identifiziert werden, die in allen Laccasen vollständig konserviert waren. Unter Berücksichtigung degenerierter Codons wurden diese Peptide in DNS zurück übersetzt, um degenerierte Primer herzustellen. Die Primer hatten die folgenden Sequenzen:

Primer E und F enthielten im 5'-Bereich eine Bam HI Schnittstelle (unterstrichen) und daran angeschlossen jeweils die entsprechende degenerierte Laccasesequenz.
PCR-Amplifikationen wurden entsprechend dem Stand der Technik nach den Angaben des Herstellers (PCR Kit von Perkin Elmer) durchgeführt: In einer ersten PCR wurden 200 ng chromosomaler T. versicolor DNS in einer 100 µl PCR eingesetzt, die den vom Hersteller bereitgestellten Puffer enthielt und darüberhinaus 1,25 U Taq Polymerase, 1,25 mM MgCl₂, je 0,2 mM der vier dNTPs (dATP, dCTP, cGTP, dTTP) und jeweils 100 pmol der Primer C und D. Die weiteren Bedingungen zur spezifischen Amplifikation des gewünschten PCR-Produkts waren: 5 min bei 94°C, gefolgt von 7 Zyklen von 0.5 min bei 94°C, 1 min bei 40°C und 2.5 min bei 60°C sowie 30 Zyklen von 0.5 min bei 94°C, 1 min bei 50°C und 2.5 min bei 72°C. 1 µl der ersten PCR wurden in einer zweiten PCR eingesetzt, die darüberhinaus 1,25 U Taq Polymerase, 1,25 mM MgCl₂, je 0,2 mM der vier dNTPs und jeweils 100 pmol der Primer E und F enthielt. Die weiteren Bedingungen zur spezifischen Amplifikation des gewünschten PCR-Produkts waren: 5 min bei 94°C, gefolgt von 7 Zyklen von 0.5 min bei 94°C, 1 min bei 40°C und 2.5 min bei 60°C sowie 30 Zyklen von 0.5 min bei 94°C, 1 min bei 50°C und 2.5 min bei 72°C. Es wurde ein PCR-Produkt von ca. 1,1 kb erhalten. Das PCR-Produkt wurde durch Agarose Gelelektrophorese gereinigt, mit dem Restriktionsenzym Bam HI geschnitten und in einen mit Bam HI geschnittenen pUC18 Vektor kloniert und in *E. coli* transformiert. Aus der Anzucht transformierter E. coli wurde das Plasmid isoliert. DNS-Sequenzanalyse vom 5'- und 3'-Ende bestätigte, daß es sich bei dem klonierten DNS-Fragment um das Fragment eines Laccasegens handelte.

Zur Vorbereitung der DNS-Sonde für das Screening von Laccasegenen wurde das Laccase-spezifische PCR-Fragment durch Behandlung mit Bam HI ausgeschnitten, über Agarose Elektrophorese isoliert und mit α-[³²P]-dATP radioaktiv markiert ("Random Priming" Kit, Boehringer Mannheim). Freie Radioaktivität wurde durch Chromatographie über Sephadex G25 (Pharmacia) abgetrennt. Die spezifische Aktivität der radioaktiv markierten DNS-Sonde betrug 1 x 10⁷ cpm/µg DNS.

### C: Isolierung chromosomaler Laccasegene.

Es wurde die im Abschnitt A beschriebene chromosomale Genbank von Trametes versicolor TV-1 verwendet. Das Screening nach genomischen Laccase Genen wurde nach dem Stand der Technik durchgeführt. In einer ersten Screeningrunde wurden Zellen von E. coli XL-1 Blue MRF' auf 10 Petrischalen zuerst kultiviert und dann mit 50000 Phagen der chromosomalen Genbank (5 - 20 kb Fraktion, siehe Beispiel 4A) pro Petrischale infiziert. Nach Inkubation bei 37°C über Nacht wurden die neu gebildeten Phagen auf Nylonfilter (Stratagene) transferiert. Die Filter wurden dann entsprechend den Empfehlungen des Herstellers mit der radioaktiv markierten Laccase-spezifischen Sonde (siehe Abschnitt B) hybridisiert. Die Hybridisierungstemperatur betrug 45°C in einem Hybridisierungspuffer mit 50% Formamidgehalt. Positive Klone wurden gepickt und durch Wiederholung des Screeningverfahrens gereinigt. Nach drei Runden der Vereinzelung wurden bei dem Screening 20 stark hybridisierende Phagenklone isoliert, die nach einer Vorschrift des Herstellers (Stratagene) durch "*in vivo* excision" in den pBK CMV Vektor (Stratagene) umkloniert wurden. Analyse der Klone durch Verdau mit Restriktionsendonucleasen und DNS-Sequenzierung ergab, daß es sich bei 15 der 20 Klone um Laccaseklone handelte. Es wurden zwei verschiedene Laccaseklone erhalten. Die beiden Laccasegene erhielten die Bezeichnung Laccase I und Laccase III. Die Plasmide mit den beiden Laccasegenen wurden als pLac100 (SEQ ID No: 1) und pLac300 (SEQ ID No: 2). bezeichnet. Beide Laccasegene enthielten neben der Sequenzinformation für das Strukturgen (kodierender Bereich) auch Sequenzinformation in der Region 5' vor dem ATG-Startkodon (Promotorbereich) sowie Sequenzinformation in der Region 3' nach dem Stopkodon (Terminatorbereich). Es handelt sich hierbei um neue genetische Regulationselemente für Trametes versicolor, die für die Herstellung von DNS-Vektoren zur Genexpression in filamentösen Pilzen aus der Klasse der Basidiomyceten verwendet werden können. Tabelle 2 gibt einen Überblick über die entsprechenden Sequenzbereiche in SEQ ID No: 1 (Laccase I Gen) und SEQ ID No: 2 (Laccase III Gen).

**Tabelle 2**

| Genetische Elemente in SEQ ID No: 1 und SEQ ID No: 2 | | |
|---|---|---|
| | SEQ ID No: 1 Laccase I Gen | SEQ ID No: 2 Laccase III Gen |
| Promotor | bp 1 - 1192 | bp 1 - 547 |
| Strukturgen (mit Introns) | bp 1193 - 3312 | bp 548 - 2542 |
| Terminator | bp 3313 - 7986 | bp 2542 - 5762 |

### 5. Beispiel

### Klonierung des T. versicolor GAPDH-Gens

### A. Isolierung einer GAPDH DNS-Sonde aus Trametes versicolor

Es wurde der Stamm Trametes versicolor TV-1 (siehe 1. Beispiel) verwendet. Anzucht von TV-1 und Isolierung von chromosomaler DNS erfolgte wie im 1. Beispiel, bzw. 4. Beispiel beschrieben.
Anhand der publizierten DNS-Sequenzen des GAPDH-Gens aus Phanerochaete chrysosporium (M. C. Harmsen et al. (1992), Curr. Genet. 22, 447 - 454) und des GAPDH-Gens aus Coriolus hirsutus (JP 9-47289) wurden Primer zur PCR-Amplifikätion eines GAPDH-spezifischen Genfragments konstruiert. Die Primer hatten die folgenden DNS-Sequenzen:

PCR-Amplifikationen wurden entsprechend dem Stand der Technik durchgeführt: In einer PCR wurden 200 ng chromosomaler Trametes versicolor DNS in einer 100 µl PCR eingesetzt, die den vom Hersteller bereitgestellten Puffer enthielt und darüberhinaus 1,25 U Taq Polymerase, 1,25 mM MgCl₂, je 0,2 mM der vier dNTPs (dATP, dCTP, cGTP, dTTP) und jeweils 100 pmol der Primer G und H. Die weiteren Bedingungen zur spezifischen Amplifikation des gewünschten PCR-Produkts waren: .5 min bei 94°C, gefolgt von 7 Zyklen von 0.5 min bei 94°C, 1 min bei 47°C und 1 min bei 72°C sowie 30 Zyklen von 0.5 min bei 94°C, 1 min bei 50°C und 1 min bei 72°C. Es wurde ein PCR-Produkt von ca. 0,43 kb erhalten.
Das PCR-Produkt wurde durch Agarose Gelelektrophorese gereinigt, in den Vektor PCR-Script SK(+) (Stratagene) kloniert und in E. coli transformiert. Aus der Anzucht transformierter E. coli wurde das Plasmid isoliert. DNS-Sequenzanalyse vom 5'- und 3'-Ende bestätigte, daß es sich bei dem klonierten DNS-Fragment um das Fragment des P. chrysosporium GAPDH-Gens handelte.

Zur Vorbereitung der DNS-Sonde für das Screening von Laccasegenen wurde das Laccase-spezifische PCR-Fragment durch Behandlung mit Not I und Eco RI ausgeschnitten, über Agarose Elektrophorese isoliert und mit α-[³²P]-dATP radioaktiv markiert ("Random Priming" Kit, Boehringer Mannheim). Freie Radioaktivität wurde durch Chromatographie über Sephadex G25 (Pharmacia) abgetrennt. Die spezifische Aktivität der radioaktiv markierten DNS-Sonde betrug 1 x 10⁷ cpm/µg DNS.

### B: Isolierung eines chromosomalen GAPDH-Gens aus T. versicolor:

Es wurde die im Abschnitt A des 4. Beispiels beschriebene chromosomale Genbank von Trametes versicolor TV-1 verwendet. Das Screening nach dem chromosomalen GAPDH-Gen wurde nach dem Stand der Technik durchgeführt. In einer ersten Screeningrunde wurden Zellen von E. coli XL-1 Blue MRF' auf 10 Petrischalen zuerst kultiviert und dann mit 50000 Phagen der Genbank (5 - 20 kb Fraktion, siehe 4. Beispiel) pro Petrischale infiziert. Nach Inkubation bei 37°C über Nacht wurden die neu gebildeten Phagen auf Nylonfilter (Stratagene) transferiert. Die Filter wurden dann entsprechend den Empfehlungen des Herstellers mit der radioaktiv markierten GAPDH-spezifischen Sonde (siehe Abschnitt A) hybridisiert. Die Hybridisierungstemperatur betrug 58°C. Die Waschtemperatur betrug 58°C. 28 positive Klone wurden gepickt. Von diesen wurden 10 Klone durch Wiederholung des Screeningverfahrens gereinigt. Nach drei Runden der Vereinzelung wurden bei dem Screening 9 stark hybridisierende Phagenklone isoliert, die nach einer Vorschrift des Herstellers (Stratagene) durch "in vivo excision" in den pBK CMV Vektor (Stratagene) umkloniert wurden. Analyse der Klone durch Verdau mit Restriktionsendonucleasen und DNS-Sequenzierung ergab, daß es sich bei allen neun Klonen um GAPDH-Klone handelte. Anhand einer Restriktionsanalyse konnten die neun Klone in 6 Klassen eingeteilt werden. Vom Klon mit dem größten GAPDH-Genfragment, bezeichnet als pGAPTV (SEQ ID NO: 3), wurde die DNS-Sequenz des verfügbaren kodierenden Bereiches (SEQ ID NO: 3, bp 1543 - 2387) und ca. 1,5 kb der Promoterregion 5' vor dem ATG Startkodon (SEQ ID NO: 3, bp 1 - 1542) bestimmt.

### 6. Beispiel

### Funktionelle Verknüpfung des GAPDH-Promotors mit dem Gen der Laccase III

### A: Klonieren des Laccase III Gens in den pBluescript Vektor

Für die weitere Bearbeitung wurde das Laccase III Gen aus pLac300 in den Vektor pBluescript umkloniert. Dazu wurde das Laccase III Gen als 3,5 kb Spe I - Sma I Fragment aus dem im 4. Beispiel erhaltenen pBK CMV Vektor isoliert und in den mit Spe I und Sma I vorgeschnittenen pBluescript Vektor subkloniert. Das dabei entstandene 6,5 kb Plasmid wurde pLac301 genannt. pLac301 enthielt zur Verknüpfung mit dem GAPDH-Promoter eine, vom pBluescript stammende Not I Schnittstelle, über die mit dem 3'-Ende des GAPDH-Promoters eine funktionelle Verknüpfung erstellt werden konnte (siehe Abschnitt B dieses Beispiels).

### B: Funktionelle Verknüpfung des ATG Translationsstartkodons des Laccase III Gens mit dem GAPDH-Promotor

Die 1,5 kb lange Promotorregion des im 4. Beispiel isolierten GAPDH-Gens wurde aus pGAPTV (SEQ ID NO:3, siehe 5. Beispiel) als Not I - BspLU11 I Fragment isoliert. Dabei stammte die Not I Schnittstelle aus dem pBK CMV Vektoranteil und die BspLU11 I Schnittstelle vom Start ATG-Kodon des GAPDH Gens.

Vektor pLac301 wurde mit Not I und partial mit BspLU11 I geschnitten und das 6 kb große Vektorfragment, das um den 0,55 kb großen Promotoranteil des Laccase III Gens verkürzt worden war, isoliert.

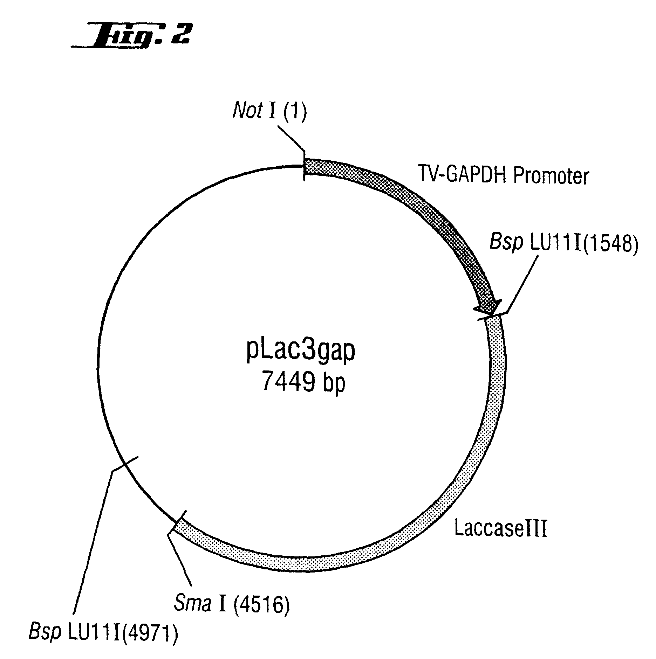

Das 1,5 kb lange Not I - BspLU11 I GAPDH Promotorfragment wurde mit dem 6 kb großen Not I - BspLU11 I Vektorfragment von pLac301 ligiert und in E. coli transformiert. Aus der Anzucht transformierter E. coli wurde das Plasmid isoliert. Positive Klone wurden durch Restriktionsanalyse und DNS-Sequenzierung überprüft. Der 7,5 kb lange Vektor, in dem das Laccase III Strukturgen funktionell mit dem Promotor des GAPDH-Gens verknüpft worden war, wurde pLac3gap (Fig. 2) bezeichnet. In pLac3gap war die GAPDH-Promotorregion über eine BspLU11 I Schnittstelle funktionell mit dem Translationsstart ATG-Kodon des Laccase III Gens verknüpft.

### C: Einbau des Selektionsmarkergens pyr G in den Vektor pLac3gap

Zur Vorbereitung wurde in den Vektor pSCpyrG in die vom Polylinker des pCR-Script SK(+) Vektors stammende Eco RI Schnittstelle eine zusätzliche Not I Schnittstelle eingebaut. Dazu wurde der Adaptor A mit der folgenden Sequenz verwendet.

Vektor pSCpyrG wurde mit Eco RI linearisiert und mit alkalischer Phosphatase 5'-dephosphoryliert. Anschließend wurde der linearisierte pSCpyrG Vektor mit verschiedenen Konzentrationen des Adaptors A ligiert und in E. coli transformiert. Positive Klone wurden durch Verdau der isolierten Plasmid DNS mit Not I identifiziert, wobei durch Einführung einer zweiten Not I Schnittstelle das 1,6 kb große Fragment des Schizophyllum commune Genfragments freigesetzt wurde. Dieser Vektor wurde pSCpyrG-Not genannt.

Der Vektor pSCpyrG-Not wurde mit Not I behandelt und das dabei freigesetzte 1,6 kb große pyr G Fragment durch Agarose Gelelektrophorese isoliert.

Der Vektor pLac3gap wurde mit Not I linearisiert und das 7,5 kb große Fragment anschließend mit alkalischer Phosphatase behandelt, um die 5'-Phosphatgruppen abzuspalten.

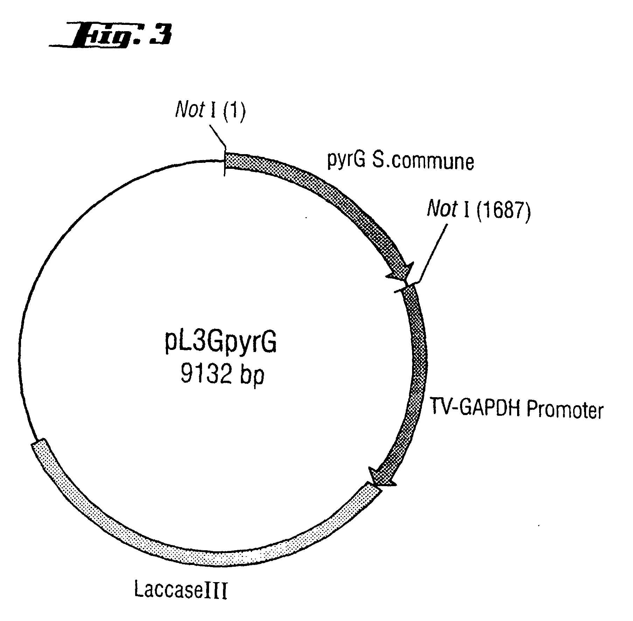

Das 1,6 kb große pyr G Fragment wurde in den mit Not I linearisierten und dephosphorylierten pLac3gap kloniert, in E. coli transformiert und von positiven E. coli Transformanten die Plasmid DNS isoliert. Positive Transformanten enthielten das 1,6 kb pyr G Genfragment. Durch Restriktionsanalyse wurden Klone identifiziert, bei denen die Orientierung des pyr G Selektionsmarkergens gleich wie die des Laccase III Gens war. Der Vektor von 9,1 kb Länge, der neben dem Laccase III Gen auch das pyr G Selektionsmarkergen enthielt, wurde pL3GpyrG (Fig. 3) bezeichnet.

### 7. Beispiel

### Überproduktion von Laccase III in Trametes versicolor

### A. Transformation von T. versicolor

Protoplasten von T. versicolor wurden nach dem in Beispiel 1 beschriebenen Verfahren hergestellt und mit dem im 6. Beispiel beschriebenen Vektor pL3GpyrG transformiert.

Wie im 2. Beispiel beschrieben, wurden Protoplasten des pyr G-defizienten Stammes Trametes versicolor F2 100B11 hergestellt und in einer Endkonzentration von 10⁸/ml suspendiert. In Inkubationsgefäßen von 12 ml Volumen wurden 0.1 ml Aliquots der Protoplasten mit 10 µg DNS des Plasmids pL3GpyrG und 30 min auf Eis inkubiert. Danach wurde langsam und unter wiederholtem mischen 1,25 ml einer PEG4000 Lösung zum Transformationsansatz gegeben. Nach weiteren 20 min Inkubation bei Raumtemperatur wurden die Reaktionsgefäße mit dem im 2. Beispiel beschriebenen OMT-Medium aufgefüllt, gemischt und 10 min bei 4°C bei 2000 x g zentrifugiert. Die Pellets wurden resuspendiert und auf osmotisch stabilisiertem MM ohne Uridin (beschrieben im 2. Beispiel) ausplattiert. Die Platten wurden bei 28°C für 14 Tage inkubiert und auf Wachstum von Kolonien überprüft. Bei verschiedenen Experimenten wurden Transformationsraten von 0,5 - 3 Transformanten/µg Plasmid DNS erzielt.

### B: Reinigung der Transformanten

Mycel der erhaltenen Transformanten wurde gepickt und durch ausplattieren auf frische Platten mit MM-Selektionsmedium ohne Uridin gereinigt. Dabei wurde das Inokulum punktförmig in der Mitte der Platte aufgebracht. Nach Inkubation für 7 bis 14 Tagen bei 28°C konnte radiales Mycelwachstum beobachtet werden. Dieser Reinigungsvorgang wurde wiederholt, wobei das Mycel für das Inokulum vom Rand der ersten Reinigungsplatte entnommen wurde. Selektivplatten wurden dann erneut mit Inokulum der zweiten Reinigungsplatte inokuliert und nachdem die Platten vollständig mit Mycel bewachsen waren, wurde die Laccaseproduktion in Schüttelkolbenanzuchten überprüft.

### C: Anzucht im Schüttelkolben

Für die Anzucht im Schüttelkolben wurden 2 cm² Mycel aus einer voll bewachsenen Platte ausgestochen, steril zerkleinert und damit eine Vorkultur von 50 ml (in einem 250 ml Erlenmeyerkolben) Malzextrakt-Medium (siehe 1. Beispiel) beimpft. Die Vorkultur wurde 6 Tage bei 28°C unter Schütteln bei 120 rpm inkubiert. Am sechsten Tag wurde die Vorkultur mit einem Ultra Turrax für 30 sec bei 9500 rpm homogenisiert und damit 250 ml Hauptkulturmedium (Zusammensetzung siehe MM im 1. Beispiel) in einem 11 Erlenmeyerkolben beimpft. Die Hauptkultur wurde dann wieder bei 28°C unter Schütteln bei 120 rpm inkubiert. Die Laccaseproduktion wurde ab dem zweiten Tag der Anzucht täglich gemessen. Laccaseaktivität wurde photometrisch mit dem Substrat ABTS (2,2'-Azino-bis(3-Ethyl-Benzthiazolin-6-Sulfonsäure)) bei 420 nm gemessen. Extinktionskoeffizient von ABTS bei 420 nm ε₄₂₀: 3,6 x 10⁴ [lx Mol⁻¹ x cm⁻¹]. Dabei entsprach 1 U Laccaseaktivität dem Umsatz von 1 µmol ABTS/min bei 37°C und einem pH von 4,5. Das Maximum der Laccaseproduktion in Schüttelkolbenanzuchten wurde 8 - 10 Tage nach Beginn der Hauptkultur erreicht. Tabelle 3 zeigt einen Vergleich verschiedener Transformanten mit dem nicht transformierten Ausgangsstamm Trametes versicolor F2 100. Für den nicht transformierten Stamm F2 100 wurde darüberhinaus die Laccaseproduktion nach Induktion mit dem in der Literatur beschriebenen Induktor 2,5-Xylidin (Yaver et al., Applied and Environmental Microbiology (1996) 62, 834 - 841) bestimmt. Wie aus Tabelle 3 zu ersehen, ist die Laccaseproduktion im Schüttelkolben bei den besten Transformanten des Stammes F2 100 gegenüber dem nichttransformierten Ausgangsstamm um den Faktor 12 (ohne Induktion), bzw. um den Faktor 5 (mit Induktion) erhöht.

**Tabelle 3**

| Stamm Trametes versicolor Maximale Laccaseproduktion (U/ml) | |
|---|---|
| F2 100 | 3,8 |
| F2 100/Xylidin* | 9,6 |
| TV Lac3gap-2 | 31,2 |
| TV Lac3gap-3 | 36,7 |
| TV Lac3gap-6 | 45,8 |
| TV Lac3gap-8 | 22,3 |
| TV Lac3gap-11 | 46,1 |
| TV Lac3gap-15 | 15,1 |
| TV Lac3gap-17 | 42,9 |
| TV Lac3gap-21 | 30,6 |
| TV Lac3gap-25 | 18,3 |

| | |
|---|---|
| * Die Induktion erfolgte drei Tage nach Beginn der Hauptkultur durch Zugabe von 2,5-Xylidin (1,5 mM Endkonzentration). | |

## Patentansprüche

1. Expressionssystem für die Produktion eines Proteins in einem filamentösen Pilz bestehend aus
a) einem Wirtsorganismus ausgewählt aus den Gattungen Trametes und Polyporus sowie
b) einem DNS-Vektor, der ein Selektionsmarkergen enthält, welches für ein Protein kodiert das nach Transformation des Wirtsorganismus eine Selektion positiver Transformanten erlaubt und ausgewählt ist aus der Gruppe der Antibiotikaresistenzgene, die für Proteine kodieren, die die wachstumshemmende Wirkung von Antibiotika aufheben, gegen die der Wirtsorganismus nicht resistent ist, der Gene, die Proteine kodieren, die zu einer farbgebenden Reaktion befähigt sind und der Gene die einen genetischen Defekt des Wirtsorganismus (Auxotrophie) komplementieren, wobei die Expression des Selektionsmarkergens durch mindestens ein im Wirtsorganismus aktives genetisches Regulationselement kontrolliert wird und
c) einem DNS Vektor der ein Gen enthält, welches für das zu produzierende Protein kodiert wobei die Expression dieses Gens und ggf. auch die Sekretion des so produzierten Proteins durch ein im Wirtsorganismus aktives genetisches Regulationselement kontrolliert wird, wobei
der DNS-Vektor, der ein Selektionsmarkergen enthält und der DNS-Vektor der das Gen enthält, welches für das zu produzierende Protein kodiert auch als ein DNS-Vektor vorliegen können.

2. Expressionssystem nach Anspruch 1, **dadurch gekennzeichnet, daß** der Wirtsorganismus ein monokaryontischer Stamm aus der Gattung Trametes oder Polyporus ist .

3. Expressionssystem nach einem der Ansprüche 1 oder 2, **dadurch gekennzeichnet, daß** der Wirtsorganismus Trametes versicolor ist.

4. Expressionssystem nach einem der Ansprüche 1 bis 3, **dadurch gekennzeichnet, daß** der Wirtsorganismus einen Defekt im Orotidin-5'-Phosphat Decarboxylasegen (pyr G-Gen) hat und für Uridin auxotroph ist und das Selektionsmarkergen ein pyr G-Gen ist.

5. Expressionssystem nach einem der Ansprüche 1 bis 4, **dadurch gekennzeichnet, daß** das im Wirtsorganismus aktive genetische Regulationselement ausgewählt ist aus der Gruppe der Promotorund ggf. Terminatorelemente für ein Glycerinaldehyd-3-Phosphat Dehydrogenasegen (GAPDH Gen), und der Promotor- und Terminatorelemente für ein Laccase Gen.

6. Expressionssystem nach einem der Ansprüche 1 bis 5, **dadurch gekennzeichnet, daß** das zu exprimierende Protein eine Laccase ist.

7. In Trametes oder Polyporus aktives Regulationselement welches **dadurch gekennzeichnet ist, daß** es den in SEQ ID NO: 1 enthaltenen Sequenzabschnitt von Base 1 - 1192 oder den in SEQ ID NO: 2 enthaltenen Sequenzabschnitt von Base 1 - 547 oder den in SEQ ID NO: 3, enthaltenen Sequenzabschnitt von Base 1365 - 1542 sowie zu diesem Sequenzabschnitt homologe Sequenzen mit einer Homologie größer als 73 % umfaßt.

8. Verfahren zur Herstellung von Pilzstämmen, die zur effizienten Expression und Sekretion von Proteinen befähigt sind **dadurch gekennzeichnet, daß** als Wirtsstamm ein Pilz ausgewählt aus den Gattungen Trametes und Polyporus mit einem Uridin auxotrophen Gendefekt verwendet wird, welcher mittels an sich bekannter Verfahren mit einem DNS-Vektor der ein Gen zur Komplementation des auxotrophen Gendefekts im Wirtsstamm besitzt, transformiert wird und aus dem Transformationsansatz die mit dem DNS-Vektor transformierten Klone durch Selektion auf Komplementation des auxotrophen Gendefekts ausgewählt werden, wobei die Expression des Gens zur Komplementation des auxotrophen Gendefekts im Wirtsstamm durch ein genetisches Regulationselement kontrolliert wird, das im Wirtsstamm aktiv ist.

9. Verfahren zur Herstellung von Proteinen, **dadurch gekennzeichnet, daß** ein Expressionssystem nach einem oder mehreren der Ansprüche 1 bis 6 in an sich bekannten Weise zur Proteinproduktion eingesetzt wird oder daß ein Pilzstamm, der nach einem Verfahren gemäß Anspruch 8 hergestellt wurde, in an sich bekannter Art und Weise kultiviert wird.

## Claims

1. Expression system for production of a protein in a filamentous fungus consisting of
a) a host organism selected from the genera Trametes and Polyporus and
b) a DNA vector which comprises a selection marker gene which codes for a protein which, after transformation of the host organism, allows selection of positive transformants and is selected from the group of antibiotic resistance genes which code for proteins which abolish the growth-inhibiting effect of antibiotics to which the host organism is not resistant, of genes which encode proteins which are capable of a colour-forming reaction, and of genes which complement a genetic defect in the host organism (auxotrophy), where expression of the selection marker gene is controlled by at least one genetic regulatory element which is active in the host organism, and
c) a DNA vector which comprises a gene which codes for the protein to be produced, where expression of this gene and, where appropriate, also secretion of the protein thus produced is controlled by a genetic regulatory element which is active in the host organism, where the DNA vector which comprises a selection marker gene, and the DNA vector which comprises the gene which codes for the protein to be produced may also be present as a DNA vector.

2. Expression system according to Claim 1, **characterized in that** the host organism is a monokaryotic strain from the genus Trametes or Polyporus.

3. Expression system according to either of Claims 1 or 2, **characterized in that** the host organism is Trametes versicolor.

4. Expression system according to any of Claims 1 to 3, **characterized in that** the host organism has a defect in the orotidine-5'-phosphate decarboxylase gene (pyr G gene) and is auxotrophic for uridine, and the selection marker gene is a pyr G gene.

5. Expression system according to any of Claims 1 to 4, **characterized in that** the genetic regulatory element active in the host organism is selected from the group of promoter and, where appropriate, terminator elements for a glyceraldehyde-3-phosphate dehydrogenase gene (GAPDH gene), and the promoter and terminator elements for a laccase gene.

6. Expression system according to any of Claims 1 to 5, **characterized in that** the protein to be expressed is a laccase.

7. Regulatory element which is active in Trametes or Polyporus and which is **characterized in that** it comprises the sequence section from base 1 - 1192 present in SEQ ID NO: 1 or the sequence section from base 1 - 547 present in SEQ ID NO: 2 or the sequence section from base 1365 - 1542 present in SEQ ID NO: 3, and sequences homologous with this sequence section and having a homology of greater than 73%.

8. Process for producing fungal strains which are capable of efficient expression and secretion of proteins, **characterized in that** a fungus selected from the genera Trametes and Polyporus which has a uridine-auxotrophic gene defect and which is transformed by methods known per se with a DNA vector which has a gene for complementation of the auxotrophic gene defect in the host strain is used as host strain, and the clones transformed with the DNA vector are selected from the transformation mixture by selection for complementation of the auxotrophic gene defect, where expression of the gene for complementation of the auxotrophic gene defect in the host strain is controlled by a genetic regulatory element which is active in the host strain.

9. Process for production of proteins, **characterized in that** an expression system according to one or more of Claims 1 to 6 is employed in a manner known per se for protein production, or **in that** a fungal strain which has been produced by a process according to Claim 9 is cultivated in a manner known per se.

## Revendications

1. Système d'expression pour la production d'une protéine dans un champignon filamenteux, constitué
a) d'un organisme hôte choisi parmi les espèces tramète et polypore, ainsi que
b) d'un vecteur d'ADN qui contient un gène marqueur de sélection, qui code pour une protéine qui permet, après la transformation de l'organisme hôte, une sélection des transformants positifs et qui est choisi parmi le groupe des gènes résistants aux antibiotiques qui codent pour des protéines qui suppriment l'effet d'inhibition de la croissance des antibiotiques contre lesquels l'organisme hôte n'est pas résistant, des gènes qui codent pour des protéines qui sont aptes à une réaction de coloration et des gènes qui complémentent un défaut génétique de l'organisme hôte (auxotrophie), l'expression du gène marqueur de sélection étant contrôlée par au moins un élément de régulation génétiquement actif dans l'organisme hôte et
c) d'un vecteur d'ADN qui contient un gène qui code pour la protéine à produire, l'expression de ce gène et, le cas échéant, également la sécrétion de la protéine produite de cette manière étant contrôlée par un élément de régulation génétiquement actif dans l'organisme hôte,
le vecteur d'ADN, qui contient un gène marqueur de sélection et le vecteur d'ADN qui contient le gène qui code pour la protéine à produire pouvant également se trouver sous forme d'un seul vecteur d'ADN.

2. Système d'expression selon la revendication 1, **caractérisé en ce que** l'organisme hôte est une souche monocaryotique de l'espèce tramète ou polypore.

3. Système d'expression selon l'une quelconque des revendications 1 ou 2, **caractérisé en ce que** l'organisme hôte est du tramète versicolore.

4. Système d'expression selon l'une quelconque des revendications 1 à 3, **caractérisé en ce que** l'organisme hôte présente un défaut dans le gène orotidine-5'-phosphate décarboxylase (gène pyr G) et est auxotrophe pour l'uridine et que le gène marqueur de sélection est un gène pyr G.

5. Système d'expression selon l'une quelconque des revendications 1 à 4, **caractérisé en ce que** l'élément de régulation génétiquement actif dans l'organisme hôte est choisi parmi le groupe des éléments promoteurs et le cas échéant de terminaison pour un gène glycéraldéhyde-3-phosphate déshydrogénase (gène GAPDH) et des éléments promoteurs et de terminaison pour un gène laccase.

6. Système d'expression selon l'une quelconque des revendications 1 à 5, **caractérisé en ce que** la protéine à exprimer est une laccase.

7. Elément de régulation actif dans un tramète ou un polypore, **caractérisé en ce qu'**il comprend la section de séquence des bases 1 - 1192 contenue dans la SEQ ID NO. : 1 ou la section de séquence des bases 1 - 547 contenue dans la SEQ ID NO. : 2 ou la section de séquence des bases 1365 - 1542 contenue dans la SEQ ID NO. : 3 ainsi que des séquences homologues de cette section de séquence présentant une homologie supérieure à 73%.

8. Procédé pour la production de souches de champignons qui sont aptes à l'expression et à la sécrétion efficace de protéines, **caractérisé en ce qu'**on utilise comme souche hôte un champignon choisi parmi les espèces tramète et polypore avec un défaut de gène auxotrophe de l'uridine, qui est transformé par des procédés connus en soi avec un vecteur d'ADN qui présente un gène pour complémenter le défaut auxotrophe de gène dans la souche hôte et qu'on choisit à partir de la charge de transformation les clones transformés avec le vecteur d'ADN par sélection via la complémentation du défaut auxotrophe du gène, l'expression du gène pour la complémentation du défaut auxotrophe du gène dans la souche hôte étant contrôlée par un élément de régulation génétique, qui est actif dans la souche hôte.

9. Procédé pour la production de protéines, **caractérisé en ce qu'**on utilise un système d'expression selon l'une ou plusieurs des revendications 1 à 6 de manière connue en soi pour la production de protéines ou **en ce qu'**on cultive une souche de champignon, qui a été produite selon un procédé selon la revendication 8, de manière connue en soi.


### SEQUENZPROTOKOLL

<110> Consortium f r elektrochemische Industrie GmbH
<120> Expressionssystem zur Produktion von Proteinen
<130> Co9618/P
<140>
   <141>
<160> 13
<170> PatentIn Vers. 2.0
<210> 1
   <211> 7986
   <212> DNA
   <213> Trametes versicolor
<400> 1
<210> 2
   <211> 5762
   <212> DNA
   <213> Trametes versicolor
<400> 2
   tggagctcgc gcgcctgcag gtcgacacta gtggatctac ctcccgtgag aggaacgacg 60
   gcgtcgggcc tttctcacgc gcagcgccga cttcgcgcga cagccgctgg tcagggcagc 120
   acagacatct cccatcaccc agcttatacg ctcagttccg gcaccgagtc tgacgaatgc 180
   ggcccgcctg caatcccgac actgctcggg gcgcgcgatc agactttccg tgaggacgta 240
   aggtgctgtc ggccacctct cgacgctctc acgcataccg cagaattcgc gcgacgaccg 300
   cgttccaggg cccttgacag atgctgacac cggtgcaatc ttgacactgt accaaccggg 360
   taagtctcgt ccttggttct cggggactgg cgccggtcgc taccccttgg tcattcactc 420
   taccagagcg ctggcttcgc cgaggtataa aggatgttgc gcgaaaccct caacacccca 480
   actcaagccc cacttgagct tttgcgagat cctccacata ccactcacta ctttcaagtt 540
   cttcaacatg tcgaggtttc actctcttct cgctttcgtc gttgcttccc ttgcggctgt 600
   ggcccacgct ggtatcggtc ctgtcgccga cctcaccatc accaacgcag cggtcagccc 660
   cgacgggttt tctcgccagg ccgtcgtcgt gaacggcggc acccctggcc ctctcatcac 720
   cggtaacatg gttcgtctcg gcgcgcacta gcggattgta tcgttcctga cacactgttg 780
   cagggggatc gcttccagct caatgtcatc gacaacctca cgaaccacac gatgctgaag 840
   agcaccagta ttgtgagcta gtattcctcc tggaggaggc ttcattgtgc taataatcgt 900
   cgtgtccagc actggcacgg tttcttccag aagggcacca actgggccga cggtcccgcc 960
   ttcatcaacc agtgcccgat ctcatctggc cactcgttcc tgtatgactt ccaggttcct 1020
   gaccaggctg gtaagtacgg tcgttatgga gtgtactgcg cattgctaaa tcgcatggtg 1080
   aacaggcacc ttctggtacc acagtcactt gtccacgcag tactgtgatg gtctgagggg 1140
   tccattcgtt gtttacgacc cgaatgaccc ggccgccgac ctgtacgacg tcgacaacgg 1200
   taaggacgaa ttcgaaccgt aaatacccgc ttactgataa ctttccgacg aattagacga 1260
   cacggtcatt acccttgcgg attggtacca cgtcgccgcg aagctgggcc ccgcattccc 1320
   gtaagtccat gggcattgtg ctattgaatc tctcttaact gtgcatatca gtctcggcgc 1380
   cgacgccact ctcatcaacg gtaagggacg ctcccccagc acgaccaccg cggacctcac 1440
   tgttatcagc gtcactccgg gtaaacggta tgctatatct tatcttatct gatggtactt 1500
   tactgagaca tgctctagtt accgtttccg cctggtgtcc ctgtcgtgcg accccaacca 1560
   caccttcagc atcgatggcc acaacatgac gatcatcgag accgactcga tcaacacggc 1620
   gcccctcgtg gtcgactcca ttcagatctt cgccgcccag cgttactcct tcgtggtaag 1680
   tttgattcct ttcatgaggt tggtcgcaat tagtgattgt atggtcatgt agctcgaggc 1740
   caaccaggcc gtcgacaact actggattcg cgccaacccg agcttcggta acgtcgggtt 1800
   caccggcggc atcaactcgg ctatccttcg ctatgatggc gccgctgcca tcgagcccac 1860
   caccacgcag accacttcga ccgagccgct caacgaggtc aacctgcacc cgctggttgc 1920
   caccgctgtc gtatgtaata tcttccgtga ttgagcgcat cgttgctgac ttcgaccccc 1980
   acagcctggc tctcccgttg cgggtggtgt tgacctggcc atcaatatgg cgttcaactt 2040
   caatggcacc aacttcttca tcaacggcgc gtctttcacg cccccgaccg tgcctgtcct 2100
   cctccagatc atcagcggcg cgcagaacgc gcaggacctc ctgccctccg gcagcgtata 2160
   ctcgctcccc tcgaacgccg acatcgagat ctccttcccc gccaccgccg ctgcccccgg 2220
   tgcgccccac cccttccact tgcatgggca cgcgttcgcg gtcgtccgca gcgccggcag 2280
   cacggtctac aactacgaca accccatctt ccgcgacgtc gtcagcacgg ggacgcctgc 2340
   ggccggtgac aacgtcacca tccgcttccg caccgacaac cccggcccgt ggttcctcca 2400
   ctgccacatc gacttccacc tcgaggccgg cttcgccgtc gtgttcgcgg aggacatccc 2460
   cgacgtcgcg tcggcgaacc ccgtccccca ggcgtggtcc gacctctgcc cgacctacga 2520
   cgcgctcgac cccagcgacc agtaaatggc ttgcgccggt cgatgatagg atatggacgg 2580
   tgacttcgca cttgcaatac ggactctcgc ctcattatgg ttacacactc gctctggatc 2640
   tctcgtctgt cgtcggaaca aatttgtata attcgcttaa tggttgaaac aaatggaata 2700
   ttgggttatt atgcacgcat ttccctgttt gagcgatgga atgatccacg gttaaaaatg 2760
   cgttagcgta acttcaagtc gaccatgctt agctgtagtg cacttgcggt acgaggtgtt 2820
   acgctttttg cacgactcct tactactcaa ctatactcaa cgctatagct ctaggttgca 2880
   ggcagttggc gttcaatatg atggaactaa tagctccata cattctggtt ggttgtacac 2940
   tgcgtgttta ctaatcgcta caagatacat ccacttcacg aactgctatc tttgggccac 3000
   gtcgcgatct tcaccgcgct ccccgtgagc gtgaacgtgt gcgtcagctt cgcgtccgtg 3060
   tcgagcgcga gcgtgtacgt cccagggtgc acccacttct ccccatcctc atccgcgcgc 3120
   gcgatcgcgc ccagcgtcac cggcagctgc gcgaccgtgc tcccccgcgg cgcgagccca 3180
   tggatgcgcg tgtacgcgac gagcgtcttc ttcgggtgcg gcgcgggccc aaaggagcca 3240
   ctcacgaaca gcagcgcgac gtagtcggac gcgaccttcc ccgtgttcgt cacgcgcacc 3300
   gcgaacgtgt cgaggggcgc caggtccagg aacgccacgc tctcctggcc gtgcgtcacc 3360
   agctgcgaga tggagtacga tttcgcgggg ccgccgaacg acgcggtgga gtcggcgcca 3420
   ggggcggccc aggcgaacgc gaaggttgtg tagtgcagcc cgaagccgaa ctcgaagact 3480
   ggggtgccgg agtaccactt gtacgtgcgc ccggggttcg tcgcgctcgg gcgcagagtc 3540
   atatccgtca tcgggacctg ttcggcgtac gcggcagggt actgcgtgat gggtaggcgt 3600
   cccgcggggg ccgccttgcc cgtgaggatg tcgaagagcg cggtgccgcc gctctggccg 3660
   gggtagccgc cccagatgat tgcgtttacc tggtggagta gatgtgcacc cgcgtgatta 3720
   gctcctgtcg cgggcgtagt tacaagcaaa agggaatgga atggtacgca ctgctttact 3780
   gtgcttgagg gcggtgtcgt cgagctggcc gccaccaaac tgcgcgacga tcagcggctt 3840
   gcctacgcgc tctagttccg cgacgaggtc gagttggttt cctggccagg ttacgttgag 3900
   gcggtctatc tcctcgcgct cgaccgtttc gtcgagccct cccgcgaaga ccaccgcgtc 3960
   cgcgcgcttc gcggcagcga cggcggctgc aaagccgctc gtgtcgttgc gggtagtcac 4020
   gttggtgccg aatacatact ccacctcaaa tcctgcttgc tgggcacctt gtactgggct 4080
   cacgaggtaa ggggcgatac cgaagtagtt gccctgcatg agccgcgtgg cgttcgccca 4140
   ggggccgatg agcgcaagct tgcggacgcg tttcgacagc gggaggagcc cgtcgttctt 4200
   cagcaggacc atgccctcga cggcagcggt atgtgcgagc tgctgagctt gcggagtgtt 4260
   cacgtcaggc catcctagct gacggtaagg ttgtgccgct ggatcgtcga agtagccgag 4320
   gctgcagagt tcgtttagaa tagatttcgg gcataacaga aggtcactta ccggacgagc 4380
   gatgcgtatt ggcggatagc tgctctacgg aggtccgtcg agttgaccaa accccgttgc 4440
   agcgcctcgg gaagataggt ggacgagaat gtcccacagt cgatgtcagt tcctgcgagc 4500
   agcgcgtccg ccgccgcttg cgccgggtcg gtggtatagt tgtgcggcgt gaaaatgttt 4560
   tgcacagcgt cgcagtcgct cgtaacccac cgattgtccg taaagcccca gtggtcgcgg 4620
   agcacgtcct gcagcaggaa gctgttcgca cacgacggga tgccgttcac ggcgttgtac 4680
   gagcacatca cgctcgcgac cttcgcgtcg cgcacgcacg tctggaacgg cgggaggtag 4740
   aactcggaca ggtcctgctg cgagacgacc gcgttgaacc cgtagcgcac gacgccctcc 4800
   cagttgtcca tgtcgtacgc ggcgaagtgc ttgcagtcgg cgacgacctt gaagtacggc 4860
   ttcgggtcga gcccgccttg caggcccagg atgaggttgt agacatattg ggaaaggtgg 4920
   aacggatcct ctcccggggt ctcctggccg cggccccatc ggggatcctt gaacgctatt 4980
   agacgctgtt tggtgagcgg tcccacaacg gagagtagaa gagaagactc acggttgata 5040
   ttgggcgtcc aataatcgag accagcgcgg ccgacgttgt tgaaggcacg ccattctgtg 5100
   ctcacgatgg tcgcgatcgc ttgaatgagt gggtcatcga acgcggcacc catcagaatg 5160
   gtttggggga aggaggtcgc ataactgaaa ttgccggatg gtgcaaaggt cacacccggg 5220
   ctctgggcta caccgtgcta aacataaaaa tgagtatatt gcgacataga taggccgaga 5280
   tacgtaccaa accttctgac caccagttgt aggcgggaag acctagacgg ggtacacccg 5340
   gagaggcgtt gactgtgttg ttagtgagct cctcgtcagt ccagatgcta atgagcgccg 5400
   tagctcgagt gatagggtcc ttggtgacat cacagacggc gttgttcttc agcgggccgt 5460
   tgacacaatc cgggaagcca tacgcccgaa cggtcgcagc gccgacggag aacgcggtga 5520
   gaaacgcgtg gagacgacgc agaccgttcg ctatctagac aggcaggaac gtgaggagac 5580
   agtccaccac gaaccgttcc attttgccct ctcttttaac ggggaactgg gcgttacgtg 5640
   ccagggagaa cgtcggagct gggggcgaag agcatgatcc aaagaattca aaaagcttct 5700
   cgagagtact tctagagcgg ccgcgggccc atcgattttc cacccggtgg gtaccagtaa 5760
   tt 5762
<210> 3
   <211> 2387
   <212> DNA
   <213> Trametes versicolor
<400> 3
   cagtatgtga agcctctggg gattcctgta gaatcatatg tgaggaaccg acgagcttcc 60
   catgcttcca ccgcatatcg tgtagtctcc taccaagcag taaccctaac ggctctttcg 120
   gtacaccgta cggagtgcga cggaaaggtc gcggacgtag tgagagaaac gatatgttca 180
   caagacctat atcatgagaa caggaacgct agagtcgtcg gccgaaaaca agtcgggtat 240
   ccgtgaggca gatatcacgt tgaccaggtt tgcgttggtg gcggatgttg cgacggtttg 300
   tacgtaccgt acatggcgta caagacgcgg gagtgagccg tgcaaaatgg gacgaatagg 360
   aaccccgtag ccgatatgga ccgcaccgga gggattgttg cccatacata agccatatgt 420
   tctcgccgtg ccgcgccgag agctggtgtg cgcactagtg ggtgctatta gaagccgagc 480
   gaggtaccgt ccctcaggcc aattggggaa taggacggag gcattcgagt aaaatacaca 540
   tgggcatgcc tggatggagg catgcacatg cgcaggaggt tagtgcagcg cggccaatag 600
   gagggcagga cggagtgaga tcaggagcga ctctggacgg gccgaagagt gagcgagagc 660
   agaggcgcgg tgtggccgca cctgatgtta gagcaaagct tatttctgaa ctcgcggctc 720
   gtggaggcca gacaggagta cgagcataga caggcgagag cggccggcga ggcgtcgagg 780
   tcgggcggag atcacgtcca tccgtccatt ctgtctgcag cctgtgctta caggcaccgg 840
   gacagaggcg tacgatgacg tcgtatcgga cggaggagcg cacgaaacgg agcgcggatc 900
   ttgatgtttg cgttagagag cagcgggatg agcgaggacg agcgttgaca aacgtctgac 960
   tgagcagggc aagcaagagc tccgtgctgc gcggcggtgg gagggaagag ccgagggcgt 1020
   gattggacga gggaaggagt catccagacc gcgtcttcgg cgaactgggc gaagaaggct 1080
   ggaagagcga cgaggctcgc cggcgcgcgc gcacgctggg ctgctatcga ttgatggtcg 1140
   ctcgagcagc gacgttggcc gaggcactct cctcgaaaga ctcaaatcgt acggtggccc 1200
   ggacatggcc taccggcgac caatccgcga tgggcaaatg tcggtctcgt tatccacgca 1260
   cgttctatct cgcccgttgg cacgcccaag gacgactgcg ctgcgactgg gcagtcgtcg 1320
   tgggggaaaa cactgcgagg tgcgcggaag ggtgtcgaag gccaaggacg agcggagaac 1380
   gggcggcggc gcgggccagc gagcgcccaa agcggaagga cagcacaggc gctgggcggg 1440
   tagcgtccga tccatctcag ataagaatcg cccctgcggt atataacgca gaccttgcgc 1500
   cccgactgaa ccccatcctc tcatcccatc caccacatcc acatgtcggt cagtacccgg 1560
   ctcacccttg cagtccctgc atgcgctgac ctctccctgc agcaacaagt caacgtcgga 1620
   atcaacgggt aagatcgctc gcggacttga attcaccgca tacatcctaa tctcgcccaa 1680
   tccgcgtcct tttccagttt cggtgagcct gctccctccc tcgcttttaa tcgcgcgcat 1740
   atactcaccc attcgcaggt cgtatcggcc gtatcgtcct ccgtaatgcc ctccagcacg 1800
   gcaagatcaa tgtcgtcgct gtgaacgagt aagcgcgccc aatctgcatc gcgtaaatca 1860
   gacgcgctct aactccctgc gcgcagccct ttcatcgacc ttgagtacat ggtctacatg 1920
   ttcaagtacg actccgtcca cggccgcttc aagggccacg tcgaggtcaa ggacggcaag 1980
   ctctgggtcg agggcaagcc catcaccgtc ttccaggaga aggacgccgc caacattccc 2040
   tggggctccg ccggcgccga ctacatcgtc gagtccaccg gtgtcttcac caccaccgaa 2100
   aagtgcgcat acactcaccc aaacatggca ttttgtgact gacgcggttc ccgatcgata 2160
   gggcctctgc ccacttgaag ggcggtgcca agaaggtcat catctccgcc ccctccgccg 2220
   acgcgcccat gttcgtctgc ggtgttaacc tggactcgta cgaccccaag tacactgtcg 2280
   tgcgtgcatt ttcccatcgc gtcgcgcgag aaccccgatt tacctccgcc cgcgtgtaga 2340
   tccactagtg tcgacctgca ggcgcgcgag ctccagcttt tgttccc 2387
<210> 4
   <211> 21
   <212> DNA
   <213> Artificial Sequence
<220>
   <223> Die Beschreibung von Knstliche Sequenz: cDNS,
<220>
   <221> primer_bind
   <222> Complement((1)..(21))
<400> 4
   tccagctcga ccttgcgccg c 21
<210> 5
   <211> 21
   <212> DNA
   <213> Artificial Sequence
<220>
   <223> Die Beschreibung von Knstliche Sequenz: cDNS
<220>
   <221> primer_bind
   <222> (1)..(17)
<400> 5
   ggatccgacg tggaggagcc g 21
<210> 6
   <211> 17
   <212> DNA
   <213> Artificial Sequence
<220>
   <223> Die Beschreibung von Knstliche Sequenz: cDNS, degeneriert
<220>
   <221> primer_bind
   <222> (1)..(17)
<400> 6
   tggcayggnt tyttyca 17
<210> 7
   <211> 14
   <212> DNA
   <213> Artificial Sequence
<220>
   <223> Die Beschreibung von Knstliche Sequenz: cDNS, degeneriert
<220>
   <221> primer_bind
   <222> (1)..(14)
<400> 7
   tcdatrtgrc artg 14
<210> 8
   <211> 27
   <212> DNA
   <213> Artificial Sequence
<220>
   <221> primer_bind
   <222> (1)..(27)
<220>
   <223> Die Beschreibung von Knstliche Sequenz: cDNS, degeneriert
<400> 8
   attcagggat cctggtayca ywsncay 27
<210> 9
   <211> 27
   <212> DNA
   <213> Artificial Sequence
<220>
   <221> primer_bind
   <222> (1)..(27)
<220>
   <223> Die Beschreibung von Knstliche Sequenz: cDNS, degeneriert
<400> 9
   atacgaggat ccrtgnccrt gnarrtg 27
<210> 10
   <211> 23
   <212> DNA
   <213> Artificial Sequence
<220>
   <221> primer_bind
   <222> (1)..(23)
<220>
   <223> Die Beschreibung von Knstliche Sequenz: cDNS
<400> 10
   cgtatcggcc gtatcgtcct ccg 23
<210> 11
   <211> 23
   <212> DNA
   <213> Artificial Sequence
<220>
   <221> primer_bind
   <222> (1)..(23)
<220>
   <223> Die Beschreibung von Knstliche Sequenz: cDNS
<400> 11
   cgcccttcaa gtgggcagag gcc 23
<210> 12
   <211> 13
   <212> DNA
   <213> Artificial Sequence
<220>
   <221> misc_feature
   <222> ()..)
<220>
   <223> Die Beschreibung von Knstliche Sequenz: Adaptor cDNS, vorwaerts Strang
<400> 12
   aattcgcggc cgc 13
<210> 13
   <211> 13
   <212> DNA
   <213> Artificial Sequence
<220>
   <221> misc_feature
   <222> (1)..(13)
<220>
   <223> Die Beschreibung von Knstliche Sequenz: Adaptor cDNS, rueckwaerts Strang
<400> 13
   aattgcggcc gcg 13